# EUROPEAN PATENT APPLICATION

(11) **EP 4 574 068 A2**
(43) Date of publication of application: **25.06.2025**
(21) Application number: 25175614.4
(22) Date of filing: 21.12.2021
(51) Int. Cl.: A61B 17/86

(54) **A SYSTEM COMPRISING A FOAM STRUCTURE AND A SURGICAL FIXATION DEVICE**

(30) Priority: 30.12.2020 US 202063131955 P
(62) Divisional of application: 21216614.4
(71) Applicant: Stryker European Operations Limited, Carrigtwohill, Co. Cork T45 HX08 (IE)
(72) Inventor: KUHN, Sven Martin, 79312 Emmendingen (DE); STARK, Tobias, 79106 Freiburg (DE); RUHR, Miriam, 21075 Hamburg (DE); GUTZEIT, Stefan, 79102 Freiburg (DE)
(74) Representative: Röthinger, Rainer

(57) **Abstract**

The disclosure relates to a surgical foam structure comprising an open-porous body made of at least one biocompatible implant material, wherein the porous body is dimensioned such that it fulfills at least one of the following: the porosity is in the range of 0.81 to 0.91 %, and the average pore diameter is in the range of 1.10 to 1.41 mm. The disclosure also relates to a system comprising the foam structure and a surgical fixation device for attaching the foam structure to bone.

## Description

### Technical Field

The present disclosure generally relates to surgical implants. In particular, the present disclosure provides a system comprising a foam structure and a surgical fixation device.

### Background

Craniofacial implants for the craniomaxillofacial (CMF) region are generally made of solid, full body structures, such as from plastic, metallic or ceramic materials. However, they have certain disadvantages, such as brittle fracture and insufficient bony ongrowth or ingrowth. Bony ongrowth or ingrowth is important because it improves the mechanical stability of the implant, reduces the risk of implant rejection and further reduces the probability of infections, which lies typically at around 5 to 15%. Known implants, such as the aforementioned ceramic implants, achieve very small bony ongrowth depths of 0.2 mm or less. Other typical implant materials are polyethylene (PE), polyetheretherketone (PEEK) and titanium (Ti).

Fastening devices, such as screws, in the CMF field are typically used to fasten such implants, such as plate-shaped implants, to bone. An implant plate with an integrated bone fixation device is provided in EP 3 441 030 A1.

### Summary

There is a need for improvements in the field of surgical implants for the CMF region and other anatomical regions.

One aspect of the present disclosure is directed towards a surgical fixation device for attaching a foam structure to bone, the fixation device comprising a body, at least one bone attachment structure provided at the body, and at least one hook configured to grip the foam structure, which extends from the body at a hook angle of less than 90° (e.g., less than 85°, less than 80°, less than 75°, or even less) or more than 90° (e.g., less than 180° and more than 90°, less than 180° and more than 95°, less than 180° and more than 100°, less than 180° and more than 105°, or even more).

The hook angle and/or hook configuration may be selected such that the fixation device, or a set of fixation devices, is capable of preventing the foam structure from "falling through" a bone opening or from moving relative to a bone surface on which the foam structure is applied. It will be appreciated that the hook angle could in this case also be 90° or around 90° when the associated hook is provided with one or more barks suitable to prevent the foam structure from getting detached from the fixation device.

The fixation device may be configured to engage the foam structure only from one face or side of the foam structure. This side can be an upper or lower side (i.e., no lateral side) of the foam structure, for example a side of the foam structure, that, when implanted, is directed towards a patient's skin. As such, fixation device may only comprise one or more hooks with tips that face away from the body.

The body may be a substantially straight strut or plate from which only the at least one hook extends. The body may be flat on its side facing away from bone.

The at least one hook may extend along a single straight line from the body. The hook, or at least two or more hooks if multiple hooks are provided, may be positioned at a distal end of the body. One or more (e.g., further) hooks may be positioned spaced apart from the distal end towards an opposite, proximal end of the body. The bone attachment structure may be positioned at the proximal end of the body.

In all aspects of the present disclosure, the foam structure may be an open-porous foam, which may be an entirely or partially open-porous foam structure, either directly or indirectly via at least one other pore. In an entirely open-porous foam structure, the inner space of every pore of the foam structure is connected to the outside of the foam structure. In a partially open-porous foam structure, the inner space of at least one pore of the foam structure has no connection to the outside of the foam structure, either directly or indirectly via at least one other pore. The foam structure may be an implant foam structure, which may be a human implant foam structure, for example a craniomaxillofacial implant foam structure.

The foam structure may have a patient-specific shape (e.g., dimensioned to fill a patient-specific bone defect, for example in the skull or another CMF region). The foam structure may have a thickness equal to or (marginally) smaller/thicker than the bone surrounding the bone defect / bone opening. The foam structure may have a substantially flat or curved shape.

A fixation device as presented herein may be shaped as a claw or as an elongated hook plate, which comprise the body, which may be an elongated plate shaped body.

The foam structure in this disclosure may be comprised of multiple, such as two or three or more, foam structures, which are connected to each other, e.g., by one of the disclosed fixation devices. Likewise, the fixation device may be adapted to connect multiple, such as two or three or more, foam structures to each other. This can be achieved by providing hooks at opposite ends/sides of a fixation device, which is dimensioned to be attached to one foam structure with one portion of its hooks and to another foam structure with another portion of its hooks.

The at least one hook may be located at a side and/or at an end and/or at a center of the body, in a longitudinal extension direction of the body (e.g., from the distal end to the proximal end). The at least one hook may be sized and shaped to be inserted into corresponding porous openings of a foam structure for fixation of the fixation device to the foam structure.

The hook angle between the body and the at least one hook may be 90° or less, or less than 180° and more than 90°, as mentioned above. The hook angle may be approximately 60° or exactly 60° or approximately 120° or exactly 120°. The hook angle may be measured in parallel to longitudinal extension of the body.

The fixation device may be symmetric to a first plane, in which a main extension direction of the body lies. The center of the bone attachment structure may be located on the main extension direction of the body, while the at least one hook is located offset to the main extension direction of the body.

The at least one hook of the fixation device may be adapted to be inserted only into the top face of the foam structure, i.e., offset from the lateral ends/sides of the foam structure contacting the bone.

However, the fixation device may also be asymmetric to the first plane or any plane, in which the main extension direction lies.

The at least one hook may extend in parallel to the first plane. However, it may be angled toward or away from the first plane at a plane angle, which may be 90°, 85°, 80° or less, such as anywhere between 45° and 80°, for example approximately 60° or exactly 60°. In case multiple hooks are provided, at least some (e.g., two or more) of the hooks may define a hook row, e.g., a second plane, in which the at least one hook extends and in which its direction of elongation lies. So, a combination of hook angle and plane angle as provided above individually is also possible.

The at least one hook may be straight, arcuate, meandering and/or shaped in a zig-zag-pattern. The at least one hook may further be helical or in the shape of a corkscrew. The at least one hook may comprise a taper along its length, which may be formed by a balloon shape or a step in its circumference, i.e., in a direction orthogonal to the hook's direction of elongation. At least one hook may comprise a barb at its end facing away from the body.

The body may have a thickness of less than 3 mm, less than 2 mm, or less than 1.5 mm, such as 0.2 to 0.6 mm or such as 0.3 to 0.6 mm.

At least one of the hooks may be dimensioned such that its maximum thickness in its region to be inserted into the foam structure is equal to or less than the thickness of the body, i.e. less than 3 mm, less than 2 mm, or less than 1.5 mm, such as within the range of 0.2 to 1.41 mm, 0.2 to 0.6 mm, 0.23 to 0.6 mm or 0.3 to 0.6 mm. Further examples include a range of 0.23 to 1.41 mm or 1.10 to 1.41 mm, such as 0.23 to 1.12 mm or 1.10 to 1.12 mm, 0.23 to 1.127 mm or 1.26 to 1.27 mm, or 1.40 to 1.41 mm, and a range of 0.23 to 0.26 mm.

Additionally or alternatively, at least one of the hooks may be dimensioned such that its maximum cross-sectional area is less than 7.1 mm², less than 3.15 mm², or less than 1.60 mm², such as within the range of 0.042 to 1.56 mm². Further examples include a range of 0.042 to 1.56 mm² or 0.95 to 1.56 mm², such as 0.042 to 0.96 mm² or 0.95 to 0.96 mm², 0.042 to 1.27 mm² or 1.24 to 1.27 mm², or 1.53 to 1.56 mm². Further ranges include 0.03 mm² to 0.29 mm², 0.04 mm² to 0.29 mm² or 0.07 mm² to 0.29 mm².

The hook thickness and/or cross-sectional area may be smaller than the thickness and/or cross-sectional area of the struts in the foam structure forming the pores/cells of the foam structure. Thereby, the hook will deform before the struts of the foam structure, when the hook is inserted into the foam structure to attach the fixation device to the foam structure.

The distance between adjacent hooks may be dimensions such that it is at least 1 mm, at least 0,5 mm or at least mm 0.23 mm. For example, at least 0.24 mm, 0.25 mm or 0.26 mm. Further examples include a distance of a multiple of 0.23 mm, 0.24 mm, 0.25mm or 0.26 mm. The distance between adjacent hooks may be dimensions such that it is a multiple of the distance between adjacent pores of the foam structure located at the surface of the foam structure, to which the fixation device is to be attached. Thereby, damage to the struts of the foam structure can be reduced/avoided.

Since the pores/cells of the foam structure are formed by struts of the foam structure, the access to the pores/cells is defined by a pore/cell window, which can be of various sizes. So, the hook thickness and/or cross-sectional area may be equal to or smaller than the diameter and/or area of the pore/cell window. The distance between adjacent hooks may be dimensions such that it is a multiple of the distance between adjacent pore/cell windows.

The at least one hook may have a smaller cross-section than the body from which it extends.

The at least one bone attachment structure in any of the surgical fixation devices presented herein may comprise at least one screw opening in the body. The screw opening may have a closed (e.g., circular) or open shape. In another variant, the at least one bone attachment structure comprises one or more spikes configured to engage bone (see, e.g., EP 3 441 030)

The at least one screw opening may be sized and shaped to receive a screw for attachment of the fixation device to another solid structure. The number of screw openings may be one or there may be multiple screw openings. Alternatively, or in addition, any other type of bone attachment structure may be provided. Such a bone attachment structure may have a specific shape for a form-fit attachment, be made of a specific material suitable for melting, welding etc., or may be suitable for adhesion, for example by means of an indentation or a through-opening in the body for receiving adhesive material, or may comprise one or more spikes.

The at least one screw opening may be located at a side and/or end of the body, which is opposite of the side/end, where the at least one hook is located, and/or at the center of the body, in the body's main extension direction. The at least one screw opening may be offset from the at least one hook, in the body's main extension direction, such that it does not extend along a path that is intersected by a hook.

The fixation device may comprise multiple hooks, which extend from the body each at the same hook angle. For example, two, three, four, five, six or more hooks may be provided.

Each hook angle and/or each plane angle may be the same for all hooks. However, at least one of the hooks may have a hook angle and/or plane angle that differs from the respective hook angle and/or the respective plane angle of at least one of the other hooks.

Hooks may be located at opposite sides and/or ends of the body, or at one side/end and at the center of the body, or only at the center of the body. In these cases, at least some of the hooks may face each other, be inclined towards each other or be inclined away from each other. The provision of hooks on both sides and/or ends may provide a fixation device that is symmetric to a third plane, which is orthogonal to the first plane and the main extension direction of the body. So, the fixation device may be symmetric to the first plane and/or the third plane.

At least two of the hooks can be of at least one of different length, width, thickness, cross-sectional shape, outer shape, and material in order to achieve various degrees of fixation in different foam structures.

The multiple hooks may form at least one hook row. One or more hooks may be offset in a direction perpendicular to the hook row (e.g., towards the proximal end of the fixation device).

Multiple hooks may form one hook row, i.e., have their direction of elongation lie in the same (second) plane. This hook row/(second) plane may be orthogonal to the main extension direction of the body and to the first plane, or may be oblique thereto.

The multiple hooks may comprise three or more hooks, at least two of which are comprised by the hook row and at least one of which is offset in a direction perpendicular to the hook row.

The hooks of one/the first hook row may be as long as the hooks of another/the second hook row. Alternatively, one hook of one hook row may differ in length compared to another hook of the same one hook row and/or compared to yet another hook of a different hook row. The hooks of one hook row may have equal lengths. The hooks of the one hook row may have different lengths than the hooks from another hook row. For example, the hooks of one/the first hook row may be shorter than the hooks of another/the second hook row. Alternatively, the hooks of one/the first hook row may be longer than the hooks of another/the second hook row.

The hooks of one/the first hook row may have the same hook angle as the hooks of another/the second hook row. However, at least one hook of one hook row may differ in hook angle compared to another hook of the same one hook row and/or compared to yet another hook of a different hook row. The hooks of one hook row may have the same hook angle. The hooks of the one hook row may have different hook angles than the hooks of another hook row. For example, the hooks of one/the first hook row may have a smaller hook angle than the hooks of another/the second hook row. Alternatively, the hooks of one/the first hook row may have a larger hook angle than the hooks of another/the second hook row.

The hooks of one/the first hook row may have a smaller distance to each other compared to the hooks of another/the second hook row. A distance to each other means the shortest distance between two adjacent hooks. However, hooks of at least one hook row, such as the first hook row, may have the same distance to each other as hooks of another hook row, such as the second hook row. Hooks of one hook row, such as a hook row with three or more hooks, may have different distances to each other. The hooks may be arranged symmetrically in their hook row. Hooks of one hook row may be offset in the main extension direction to hooks of an adjacent hook row.

The multiple hooks may extend from the body at the same hook angle. As an option, each of the multiple hooks has a tip and the tips span a polygonal area.

The polygonal area may be a trapezoid area. The short base of the trapezoid may be closer to one end of the body, while the long base of the trapezoid may be closer to the other end of the body. The short and long base are the parallel sides of the trapezoid.

The at least one hook may be plastically and/or elastically deformable. The at least one hook may be plastically and/or elastically deformable in a direction orthogonal to its direction of elongation, for example more easily than the body. Further, the size, shape and/or material of the at least one hook may be adapted to promote a plastic deformation, for example caused by the intended foam structure, to which the fixation device is to be attached/fixed.

Another aspect of the present disclosure is directed towards a surgical fixation device for attaching a foam structure to bone, the fixation device comprising at least one first attachment structure configured to attach the fixation device to bone; and at least one second attachment structure configured to attach the fixation device to the foam structure. The surgical fixation device may be provided in a system with any of the foam structure realizations presented herein.

The first attachment structure may comprise a mesh. The mesh may laterally extend beyond the second attachment structure and/or the foam structure.

The first attachment structure may define one or more openings to receive bone fasteners. The openings may be provided by mesh openings or by dedicated openings separately defined in the mesh.

The first attachment structure may extend circumferentially around the second attachment structure and/or the foam structure.

The second attachment structure may comprise a mesh covering the foam structure. The covering may be either on the top or on the bottom of the foam structure. The latter could, for example, be used in bone augmentation (e.g., when the foam structure is to locally increase bone thickness).

The second attachment structure may define openings to receive fasteners (e.g., screws). The second attachment structure may comprise hooks (e.g., with barbs).

The mesh in any of the aspects of the present disclosure may comprise a pattern of dedicated structures (e.g., circular rings that define dedicated orifices, optionally for receiving bone screws) that are attached to each other via arms. Three, four or more arms may extend from each structure. Each arm may have one bend (e.g., of 20° to 90°). The mesh in any of the aspects of the present disclosure may have a configuration as disclosed in EP 0 654 250 A1.

Another aspect of the present disclosure is directed towards a system comprising any of the disclosed fixation devices, or any other fixation device, and a foam structure, the foam structure comprising a porous body made of at least one biocompatible implant material, wherein, as an option, the porous body is coated with a coating, which is capable of stimulating bony ingrowth. The fixation device is configured to attach the foam structure to bone.

The porous body and, optionally, the fixation device, may be 3D-printed, for example in a monolithic manner. The pores may be open pores for enabling bone ingrowth into the pore openings. The porous body and the fixation device may be coated with a coating, which is capable of stimulating bony ingrowth.

The foam structure may promote a deep bony ingrowth into and very often all the way through the implant foam structure, thereby creating a bony bridge across an opening in the bone. The latter is particularly important for critical size bone defects, which cannot naturally close on their own. This improves the mechanical stability of the implant in the area of the opening in the bone. It also reduces the risk of implant rejection and further reduces the probability of infections. The achievable ingrowth depth is substantially larger than possible with typical implants. Of course, the disclosed implant may be used in other areas of the human and animal body, not just the craniomaxillofacial region (e.g., as a tibia implant, a femur implant, a spinal implant such as a pedicle screw, etc.).

The biocompatible implant material of the porous body as well as the entire foam structure may be a metal, such as titanium (Ti), which in combination with the porous structure of the porous body already promotes bony ingrowth. The porous body as well as the entire foam structure may be made entirely or partially of at least one resorbable biocompatible implant material.

The coating may consist of or comprise hydroxyapatite (HA). HA has shown outstanding results in maximizing bony ingrowth, in particular when combined with a metal biocompatible implant material, such as titanium (Ti). The thickness of the coating may be 200 µm or less, such as less than 175 µm, such as less than 150 µm, such as less than 125 µm, less than 100 µm, less than 75 µm, less than 50 µm, less than 25 µm. The thickness of the coating may be more than 1 µm, such as more than 3 µm or more. Further ranges are 1 to 25 µm, 3 to 25 µm, 5 to 25 µm, 10 to 25 µm or 15 to 25 µm. The thickness of the coating may be 20 µm.

The coating may be either fully or partially provided on the foam structure. Additionally or alternatively, the coating may be such that particles of the coating material, e.g., HA, cannot detach from the coating on the foam structure, when the foam structure is bend, for example during typical use of the foam structure.

The porous body may have a patient-specific shape (which can, for example, be realized by 3D printing). The porous body may be shaped as a truncated cone. This form is advantageous in case of forces being applied from the wider side of the truncated cone, which is typically the outside of the body, in which the foam structure is placed. Also, the contact area with the surrounding bone is enlarged, which is beneficial to bony ingrowth, for example in the cranial region.

The porous body/foam structure may be monolithic and made from the same material. However, the porous body/foam structure may be manufactured by connecting/attaching several parts made of the same or different materials. The connecting/attachment means/method for this purpose may be any means/method of this disclosure or known in the prior art.

The porosity of the foam structure may be uniform, such that the size of the pores of the foam structure is uniform. However, the porosity of the foam structure may vary throughout the foam structure. For example, it may follow a specific pattern. The porosity may vary, such as decrease or increase, from a body edge of the foam structure towards the center of the foam structure.

An edge, i.e., a side, of the porous body may comprise a partially or fully closed layer. The layer may extend partially or entirely over the edge/side.

Any component of the foam structure may be made of titanium (Ti). However, components may not be made of titanium (Ti) but of a titanium alloy, comprise or consist of one or multiple other biocompatible implant materials, such as resorbable biocompatible implant materials.

The foam structure, such as all of its components, may allow drilling of holes into it and/or through it.

Further, in this disclosure, the foam structure may comprise a (e.g., 2D or 3D) printed or laser engraved marking, indicating, for example, an anatomic orientation (i.e., an alignment indication) and/or identifying the patient.

The foam structure may be adapted to be sufficiently stable to assure safety against pressure from outside and inside a patient's body, to allow slight bending to assure a good fit to or in bone, and/or to allow being cut by a mechanical or handheld electronic tool typically used by a surgeon. This can be achieved by all of the components of the foam structure provided in this disclosure.

The porous body and/or foam structure in all aspects of the present disclosure may be dimensioned such that it fulfills at least one of the following characteristics:
i. Porosity of more than 0.81 %, such as in the range of 0.81 to 0.91 %
ii. Avg Cell Volume of at least 0.69 mm, such as in the range of 0.69 to 1.47 mm³
iii. Avg Cell diameter of at least 1.10 mm, such as in the range of 1.10 to 1.41 mm

The porous body and/or foam structure may be dimensioned such that it fulfills (ii.) and (iii.), for example only for open cells/pores. The porous body and/or foam structure may be dimension such that it fulfills (i.) or a combination of (i.), (ii.) and (iii.), for example only for open cells/pores. "Open" meaning that the inner space of the cell/pore is connected to the outside of the porous body/foam structure either directly or indirectly, such as via at least one other cell/pore. The above characteristics may apply at least to a layer in the foam structure, to allow a bony ingrowth from one end/side of the foam structure to another end/side of the foam structure, which differ from each other, to thereby allow the creation of a bony ingrowth bridge through the foam structure. The above values may be set / predetermined stochastically.

The porous body may be 3D-printed. In particular, the porous body may be printed in a patient-specific shape (e.g., to fill a particular bone defect in the patient's anatomy).

The porous body and the fixation device may be a single 3D-printed monolithic structure.

The porous body may alternatively or additionally be manufactured by a different method.

The foam structure or system may further comprise an attachment structure, which is monolithic with or fixed to the porous body, wherein, as an option, the fixation device may constitute or comprise the attachment structure. The attachment structure may define one or more attachment regions (e.g., with associated attachment openings). The attachment structure may constitute or be comprised by a bone plate when it is used to attach the porous body to bone. The attachment structure may be bent (e.g., in plane or out of plane). The attachment structure may comprise one or more attachment openings (e.g., as further described below), which may be provided as through-openings in the attachment structure for inserting screws or other bone fasteners into bone and thereby attaching the attachment structure to bone.

An attachment structure may be provided at the top end/side of the porous body. The attachment structure may be monolithic and made from the same material as the porous body. In fact, the attachment structure and the porous body, which may constitute the entire foam structure, may be monolithic, i.e. one piece, and may as such be 3D-printed. Alternatively, the attachment structure may be made as a separate piece and may be made of a different material than that of the porous body. Even when the attachment structure and the porous body are separate pieces, they still may both be 3D-printed and may be fixed to each other, for example by a welding, press-fit, undercut, screwing, hooks and/or the fixation device according to this disclosure.

The attachment structure may be of titanium (Ti) and may be shaped as a plate/sheet and may further comprise an edge portion, which extends from the body edge of the foam structure. The attachment structure may also be shaped as a ring or consist of the edge portion, which is shaped as a ring. The edge portion may have a frame-like configuration. The edge portion may have a central opening. The attachment structure may be entirely made of a mesh structure and may have a mesh edge around the edge portion. The mesh edge may be open. The mesh edge may be a fully closed bar/frame. The mesh structure may be elastically and/or plastically deformable. Being plastically deformable allows the mesh structure to be adapted to a patient's natural bone surface geometry. The mesh structure, such as at least in the edge portion, may comprises holes, which are adapted to receive screws. These holes may vary in size, such that differently sized screws can be used. Additionally or alternatively, attachment openings may be provided on the edge portion. The provision of attachment openings may be omitted, when the mesh structure at least in the edge portion comprises holes, which are adapted to receive screws. Only the edge portion may be made of a solid titanium (Ti) plate/sheet.

The attachment structure may consist only of the edge portion.

The edge portion may be made entirely of a mesh structure or of the foam structure of the porous body. The edge portion may be formed by an outer section of a mesh structure and an inner section of a fully closed layer, which may be directly attached to the outer section. The inner section may extend further onto or into the porous body. Suitable mesh structures are known from EP 0 654 250 A1.

The edge portion may extend fully or partially around the foam structure.

One side of the porous body may comprise a closed layer.

The attachment structure and the closed layer may be provided on opposite sides of the porous body. The closed layer may extend partially or entirely over the side.

The foam structure or the porous body may comprise recesses on one side or on opposite sides of the porous body.

The foam structure or the porous body may comprise top recesses, which allow the reuse of existing bone chip pieces, and/or bottom recesses, which improve bendability of the foam structure. Further, recesses in the form of a spider web may be provided on either side of the porous body.

Yet another aspect of the present disclosure is directed towards a system, comprising a foam structure and either (i) a fixation device as generally defined above, or (ii) a cranial plate, capable of being attached to the foam structure, the cranial plate comprising a plate body and a plurality of spikes extending from the plate body, the spikes configured to engage bone (e.g., laterally in the region of a bone defect).

At least one hook of the fixation device may be adapted to the foam structure such that the hook deforms plastically easier than the foam structure. The at least one hook may have a length that exceeds an average pore size of the foam structure (e.g., by a factor of 1.5, 2, 3, 4 or more).

The system may comprise multiple fixation devices that are circumferentially offset relative to the foam structure.

Yet another aspect of the present disclosure is directed towards a surgical foam structure comprising: an open-porous body made of at least one biocompatible implant material, wherein the porous body, as an option, is coated with a coating, which is capable of stimulating bony ingrowth into the pores. The foam structure may be used in the context of any of the aspects of the present disclosure.

The coating may be a hydroxyapatite coating or another coating.

The coating may have a thickness between 1 µm and 200 µm, in particular between 3 µm and 100 µm.

At least more than 50% (or more than 60% or more than 70%) of the coated pores may have an internal dimension ranging between 300 µm and 1.000 µm, in particular 400 µm and 600 µm. The pores may substantially have the shape of an octaeder.

A stabilizing structure may preferably monolithically be provided on one side of the foam structure, preferably in a monolithic configuration with the foam structure. The stabilizing structure may at least partially extend into the foam structure or may (e.g., fully) be arranged on top of the foam structure. The stabilizing structure may be constituted or comprised by the fixation device. The stabilizing structure may take the form of a bone plate when it is used to attach the porous body to bone. The stabilizing structure may be bent (e.g., in plane or out of plane). The stabilizing structure may comprise one or more bone attachment regions (e.g., as an attachment structure) with one or more attachment openings (e.g., as further described below), which may be provided as through-openings for inserting screws or other bone fasteners into bone and thereby attaching the stabilizing structure to bone.

The stabilizing structure may laterally extend over the foam structure to define one or more bone attachment regions, wherein, as an option, a screw hole is formed in each bone attachment region.

The stabilizing structure may comprise a plurality of geometric entities that are at least in regions spaced apart from each other.

The geometric entities may comprise straight or non-straight (e.g., curved or bent) lines, wherein, as an option, the lines form a mesh or extend substantially parallel to each other. The mesh may comprise struts, or arms, that each extend between at least two connection points with other struts, or arms. Exemplary meshes are known from EP 0 654 250 A1.

The geometric entities may be provided over more than 60% or more than 80% of the surface of the foam structure so as to properly fulfil their stabilizing function in regard to the foam structure. If, for example, the geometric entities are defined by a mesh, the mesh may fully (100%) cover the surface of the foam structure.

The geometric entities may have at least one dimension that exceeds an average pore size and/or that exceeds 500 µm, in particular 1000 µm or 1500 µm. The average pore size being equal to or more than 300 µm, i.e. 0.3 mm, preferably, 500 µm, i.e. 0.5 mm, and/or equal to or less than 1000 µm, i.e. 1 mm. The at least one dimension may be less than 5 mm, in particular less than 3 mm or less than 1 mm. The at least one dimension may be a height dimension. The height dimension may in some configuration be defined in a direction that extends perpendicular to an adjacent bone surface.

The fixation device, optionally comprising at least one of the attachment or stabilizing structure, may be solid or mesh shaped. The attachment structure may be textured to allow bone to cover the respective surface quickly. Further, small holes of 35 µm and higher may be provided in a solid attachment structure to allow osteoblast to grow from the inside of the foam structure to cover the solid attachment structure. The foam structure may be either attached to or monolithic with the fixation device.

Parameter combinations, which have been found to synergistically improve bone ingrowth, have been exemplary tested in the following tables 1 to 4. In each table, the fixation device, optionally comprising at least one of the attachment structure and the stabilizing structure, and the foam structure are made of the same Ti grade material. Further, each table indicates different structural thicknesses of the fixation device ("structure thickness"). For each "structure thickness", different combinations of "foam thickness", i.e., the foam structure thickness, and "porosity", i.e., the porosity of the foam structure, have been successfully tested. These test results form the basis for ranges covered in this disclosure, wherein the ranges span from the lowest to the largest tested combinations of foam thickness and porosity for a specific Ti grade and structure thickness, including the maximum and minimum values.

**Table 1**

| Combinations ↓ | Titanium Grade 1 | | | | | |
|---|---|---|---|---|---|---|
| | Structure thickness [mm] | | 0.3 | 0.45 | 0.6 | 1 |
| 1 | Foam thickness [mm] | | 11.3 | 9.2 | 7.6 | 4.9 |
| | Porosity | | 1.4 | 1.4 | 1.4 | 1.4 |
| 2 | Foam thickness [mm] | | 9.7 | 7.6 | 5.9 | 3.2 |
| | Porosity | | 1.2 | 1.2 | 1.2 | 1.2 |
| 3 | Foam thickness [mm] | | 6.5 | 4.8 | 3.4 | 1.6 |
| | Porosity | | 1 | 1 | 1 | 1 |
| 4 | Foam thickness [mm] | | 5.2 | 3.6 | 2.3 | min. one average pore size |
| | Porosity | | 0.8 | 0.8 | 0.8 | 0.8 |

**Table 2**

| Combinations ↓ | Titanium Grade 2 | | | | | |
|---|---|---|---|---|---|---|
| | Structure thickness [mm] | 0.2 | 0.3 | 0.45 | 0.6 | 1 |
| 1 | Foam thickness [mm] | 7 | 5.5 | 3.7 | 3.4 | 3 |
| | Porosity | 1.4 | 1.4 | 1.4 | 1.4 | 1.4 |
| 2 | Foam thickness [mm] | 6 | 3.7 | 2.8 | 2.3 | 2 |
| | Porosity | 1.2 | 1.2 | 1.2 | 1.2 | 1.2 |
| 3 | Foam thickness [mm] | 4 | 2 | 1 | min. one average pore size | min. one average pore size |
| | Porosity | 1 | 1 | 1 | 1 | 1 |
| 4 | Foam thickness [mm] | 3.2 | 1.6 | min. one average pore size | min. one average pore size | min. one average pore size |
| | Porosity | 0.8 | 0.8 | 0.8 | 0.8 | 0.8 |

**Table 3**

| Combinations ↓ | Titanium Grade 3 | | | | | |
|---|---|---|---|---|---|---|
| | Structure thickness [mm] | 0.1 | 0.3 | 0.45 | 0.6 | 1 |
| 1 | Foam thickness [mm] | 5.1 | 3.8 | 2.4 | 2 | 1.6 |
| | Porosity | 1.4 | 1.4 | 1.4 | 1.4 | 1.4 |
| 2 | Foam thickness [mm] | 4.3 | 3.2 | 2.1 | 1.8 | 1.4 |
| | Porosity | 1.2 | 1.2 | 1.2 | 1.2 | 1.2 |
| 3 | Foam thickness [mm] | 2.9 | 1.7 | min. one average pore size | min. one average pore size | min. one average pore size |
| | Porosity | 1 | 1 | 1 | 1 | 1 |
| 4 | Foam thickness [mm] | 2.3 | 1.3 | min. one average pore size | min. one average pore size | min. one average pore size |
| | Porosity | 0.8 | 0.8 | 0.8 | 0.8 | 0.8 |

**Table 4**

| Combinations ↓ | Titanium Grade 4 | | | | | |
|---|---|---|---|---|---|---|
| | Structure thickness [mm] | 0.1 | 0.3 | 0.45 | 0.6 | 1 |
| 1 | Foam thickness [mm] | 4 | 3.3 | 2 | min. one average pore size | min. one average pore size |
| | Porosity | 1.4 | 1.4 | 1.4 | 1.4 | 1.4 |
| 2 | Foam thickness [mm] | 3.4 | 2.8 | 1.8 | min. one average pore size | min. one average pore size |
| | Porosity | 1.2 | 1.2 | 1.2 | 1.2 | 1.2 |
| 3 | Foam thickness [mm] | 2.3 | 2 | 1.4 | min. one average pore size | min. one average pore size |
| | Porosity | 1 | 1 | 1 | 1 | 1 |
| 4 | Foam thickness [mm] | 1.8 | 1.6 | min. one average pore size | min. one average pore size | min. one average pore size |
| | Porosity | 0.8 | 0.8 | 0.8 | 0.8 | 0.8 |

In the above test results, the following test results - see Tables 1.1 to 4.1 - are preferred.

**Table 1.1**

| Combinations ↓ | Titanium Grade 1 | | | | | |
|---|---|---|---|---|---|---|
| | Structure thickness [mm] | | 0.3 | 0.45 | 0.6 | 1 |
| 1 | Foam thickness [mm] | | | | | |
| | Porosity | | | | | |
| 2 | Foam thickness [mm] | | | | | 3.2 |
| | Porosity | | | | | 1.2 |
| 3 | Foam thickness [mm] | | | | 3.4 | 1.6 |
| | Porosity | | | | 1 | 1 |
| 4 | Foam thickness [mm] | | | 3.6 | 2.3 | min. one average pore size |
| | Porosity | | | 0.8 | 0.8 | 0.8 |

**Table 2.1**

| Combinations ↓ | Titanium Grade 2 | | | | | |
|---|---|---|---|---|---|---|
| | Structure thickness [mm] | 0.2 | 0.3 | 0.45 | 0.6 | 1 |
| 1 | Foam thickness [mm] | | | | 3.4 | 3 |
| | Porosity | | | | 1.4 | 1.4 |
| 2 | Foam thickness [mm] | | | 2.8 | 2.3 | 2 |
| | Porosity | | | 1.2 | 1.2 | 1.2 |
| 3 | Foam thickness [mm] | | 2 | 1 | min. one average pore size | min. one average pore size |
| | Porosity | | 1 | 1 | 1 | 1 |
| 4 | Foam thickness [mm] | 3.2 | 1.6 | min. one average pore size | min. one average pore size | min. one average pore size |
| | Porosity | 0.8 | 0.8 | 0.8 | 0.8 | 0.8 |

**Table 3.1**

| Combinations ↓ | Titanium Grade 3 | | | | | |
|---|---|---|---|---|---|---|
| | Structure thickness [mm] | 0.1 | 0.3 | 0.45 | 0.6 | 1 |
| 1 | Foam thickness [mm] | | | | 2 | 1.6 |
| | Porosity | | | | 1.4 | 1.4 |
| 2 | Foam thickness [mm] | | | 2.1 | 1.8 | 1.4 |
| | Porosity | | | 1.2 | 1.2 | 1.2 |
| 3 | Foam thickness [mm] | 2.9 | 1.7 | min. one average pore size | min. one average pore size | min. one average pore size |
| | Porosity | 1 | 1 | 1 | 1 | 1 |
| 4 | Foam thickness [mm] | 2.3 | 1.3 | min. one average pore size | min. one average pore size | min. one average pore size |
| | Porosity | 0.8 | 0.8 | 0.8 | 0.8 | 0.8 |

**Table 4.1**

| Combinations ↓ | Titanium Grade 4 | | | | | |
|---|---|---|---|---|---|---|
| | Structure thickness [mm] | 0.1 | 0.3 | 0.45 | 0.6 | 1 |
| 1 | Foam thickness [mm] | | 3.3 | 2 | min. one average pore size | min. one average pore size |
| | Porosity | | 1.4 | 1.4 | 1.4 | 1.4 |
| 2 | Foam thickness [mm] | 3.4 | 2.8 | 1.8 | min. one average pore size | min. one average pore size |
| | Porosity | 1.2 | 1.2 | 1.2 | 1.2 | 1.2 |
| 3 | Foam thickness [mm] | 2.3 | 2 | 1.4 | min. one average pore size | min. one average pore size |
| | Porosity | 1 | 1 | 1 | 1 | 1 |
| 4 | Foam thickness [mm] | 1.8 | 1.6 | min. one average pore size | min. one average pore size | min. one average pore size |
| | Porosity | 0.8 | 0.8 | 0.8 | 0.8 | 0.8 |

In other words, when the foam structure and a surgical fixation device are made of the same material, such as Ti grade 1, 2, 3 or 4, and with a thickness of the surgical fixation device of 1 mm or less, such as between 0.4 and 0.6 mm, preferably at the attachment region with the foam structure, the foam structure - according to tables 1 to 4 - comprises a thickness in the range of 11.3 mm to 0.3 mm as well as a porosity in the range of 1.4 to 0.8, preferably - according to tables 1.1 to 4.1 - a thickness in the range of 3.4 mm to 0.3 mm as well as a porosity in the range of 1.4 to 0.8. Corresponding ranges for structure thicknesses between 0.3 and 0.6 are of further interest and derivable from the above tables in accordance with the previously mentioned ranges.

Yet another aspect of the present disclosure is directed towards a method for closing an opening in a bone structure with a foam structure, comprising the following steps: providing a foam structure with substantially the dimensions of the opening; providing a fixation device or a cranial plate; attaching the fixation device or the cranial plate to the foam structure, thereby creating a system of the respective two components; inserting the foam structure of the system into the opening; and attaching the system to bone solid structure. It is to be noted that in some variants, the fixation device may at the same time constitute the cranial plate, or a portion thereon.

Attaching the fixation device or the cranial plate to the foam structure may comprise using at least one of a hammer and a screwdriver. For example, the hooks may be hammered into foam structure, or the fixation device or cranial plate may be screwed onto the foam structure.

The fixation device may also be realized as a bone screw or a bone plate. The bone plate optionally is circular, oval, ring shaped or elongated (e.g., linear). The bone plate optionally covers the foam structure partially or entirely.

At least one of the foam structure and the fixation device may comprise one or more protruding eyes. The one or more eyes may protrude in a height direction. Each eye may approximately form a semicircle in a plane that is orthogonal to the surface from which the eye protrudes. However, this shape and inclination of the eye may differ. The eye may provide an opening. Each eye and/or its opening may be shaped and dimensioned to be suitable for suturing muscle to it or for receiving a surgical wire or thread. Each eye can be a separate structure that is attached to the foam structure or integrally formed with the foam structure, e.g., by 3D-printing.

At least one of the foam structure and the fixation device may comprise at least one alignment indication. In same variants, the alignment indication helps the surgeon insert and align the foam structure properly during surgery. The indication may be provided on the foam structure, e.g., on the attachment structure and/or the porous body. In particular, the indication may be provided at an edge and/or edge portion of the attachment structure or the foam structure. Structurally, the indication may be formed as an indentation or as a protrusion to allow the surgeon to feel the indication. Further, in case the foam structure comprises several indications, at least some of those may differ from each other and be distributed, for example, on the surface of the foam structure. The indication may be an arrow, but the shape of the indication is not limited thereto and may differ.

The foam structure may comprise one or more lateral recesses. The one or more lateral recesses may be provided with or without the top recesses and/or the bottom recesses. Further, each of these recesses may be dimensioned and shaped such that it is capable of holding bone chips, for example by providing a recess that widens towards the inside direction. Thereby, bone chips can be pushed into the recess but will cant therein and become less likely to escape the recess during handling of the foam structure.

The system may be configured to be implanted in the mandibula or maxilla region (e.g., to extend over a mandibular gap) or in any long bone, such as the tibia, femur or the fibula (e.g., to cover a hole in the bone) or in the spinal region (e.g., in a vertebra). The system may comprise a foam structure with a fixation device defining an attachment structure. The fixation device with attachment structure may comprise, or constitute, a bone plate or a mesh. The fixation device may comprise an enclosure that may at least partially (e.g., over more than 100° or more than 160° or more than 180°) wrap around the porous body, which extends between two distant ends of the enclosure, optionally to contact bone, and allow bone ingrowth within the enclosure. Instead of providing the enclosure, the porous body may be directly connected with the fixation device (e.g., configured as a bone plate). All of these components may be manufactured as one single piece / monolithically, e.g., by 3D-printing, or may be made separately and connected to each other at a later stage prior to surgery by one of any known connection method, e.g., welding, screws etc. In each of these cases, i.e., the enclosure with porous body or porous body alone, there may be provided a dental implant recess. The dental implant recess may be formed as an indentation in the enclosure and/or the porous body.

The attachment structure of the fixation device, i.e., the bone plate, may have multiple branches, optionally with screw holes.

Where soft tissue retraction and implant exposure might become an issue, the attachment structure may be covered with a Medpor barrier or hydrogel (resorbable or non-resorbable) or autologous tissue (fat, bone chips).

Where infection may occur, it is possible to add antibiotics to the coating.

For not load-bearing use cases, and in other scenarios, the surgical fixation device (in particular an attachment structure comprised thereby) and the porous body may be connected by a resorbable connector (e.g., a yarn or wire).

The porous body may comprise multiple porous sub-structures, i.e., sub-bodies, which are optionally provided in a row and connected in such a manner as to be capable to be inclined relative to each other. For this purpose the sub-bodies may be portions of a monolithic porous body connected by interconnecting sections with increased flexibility compared the rest of the body. These interconnecting sections may be provided by merely weakening the otherwise uniform porous body locally. Alternatively or additionally, hinges made of the same or different material may be provided between the separately formed porous sub-bodies. Such a system may be used for spinal indications.

The surgical fixation device (e.g., in the form of a bone screw such as a spinal screw, in particular a pedicle screw) may be provided with an outer thread and may comprise a recess filled by at least a portion of the foam structure. The foam structure may extend between two thread sections of the outer thread. The foam structure may extend axially through the surgical fixation device to be exposed at one or both axial ends of the surgical fixation device. In this system, the foam structure can be ring shaped.

The fixation device (i.e., in terms of the attachment structure) and the foam structure may be custom made to correspond to a bone cavity and the outer shape of a bone with the bone cavity.

The edge portion of the attachment structure may be non-uniform and comprise a varying extension from the porous body along the circumference of the porous body.

### Brief Description of the Drawings

Further details, advantages and aspects of the present disclosure will become apparent from the following description taken in conjunction with the drawings, wherein:
- Fig. 1: shows a perspective view of a fixation device for fixation of foam structures;
- Fig. 2: shows the face of the hook of the fixation device in Fig. 1;
- Fig. 3: shows a modification of the face of the hook in Fig. 2;
- Fig. 4: shows another modification of the face of the hook in Fig. 2;
- Fig. 5: shows a perspective view of a modification of the fixation device in Fig. 1;
- Fig. 6: shows a perspective view of a foam structure consisting of a porous body;
- Fig. 7: shows a modification of the foam structure in Fig. 6, which comprises a porous body along with an attachment structure;
- Fig. 8: shows a modification of the foam structure in Fig. 7;
- Fig. 9: shows a modification of the foam structure in Fig. 8;
- Fig. 10: shows a modification of the foam structure in Fig. 9;
- Fig. 11: shows a modification of the foam structure in Fig. 10;
- Fig. 12: shows a modification of the foam structure in Fig. 11;
- Fig. 13: shows a modification of the foam structure in Fig. 11;
- Fig. 14: shows a side view of a modification of the foam structure in Fig. 6;
- Figs. 14A-C: show mesh-based systems similar to the ones of Figs. 6 to 14;
- Fig. 15: shows a table of samples with different foam characteristics;
- Fig. 16: shows a side cross-sectional view of a portion of a craniofacial bone structure with an opening to be closed and the exemplary foam structure in Fig. 6; and a first step of a method for closing an opening;
- Fig. 17: shows a system, comprising the foam structure in Fig. 6 and the fixation device in Fig. 5; and a second step of the method for closing an opening;
- Fig. 18: shows the system in Fig. 17 in a state, where the fixation device is fixed to the foam structure; and a third step of the method for closing an opening;
- Fig. 19: shows the system in Fig. 18 in a state, where the system is inserted into the opening; and a fourth step of the method for closing an opening;
- Fig. 20: shows the inserted system in Fig. 19 in a state, where the system is attached/fixed to the craniofacial bone structure with a screw passing through the screw opening; and a fifth step of the method for closing an opening;
- Fig. 20A: shows a modification of the system in Fig. 20;
- Fig. 20B: shows a top view of the modification in Fig. 20A;
- Fig. 21: shows a cranial plate, which is already known in the prior art from EP 3 441 030 A1;
- Fig. 22: shows the disengaging configuration associated with the cranial plate in Fig. 21 and a foam structure;
- Fig. 23: shows the engaging configuration associated with the cranial plate in Fig. 21 and a foam structure;
- Fig. 24: shows a perspective view of a modification of the fixation device in Fig. 1 with three hooks in a hook row;
- Fig. 25: shows another modification of the fixation device in Fig. 1 fixed to a foam;
- Fig. 26: shows a state, in which one of the fixation devices is fixed to a foam structure by means of at least one hook and fixed to a bone by means of at least one screw;

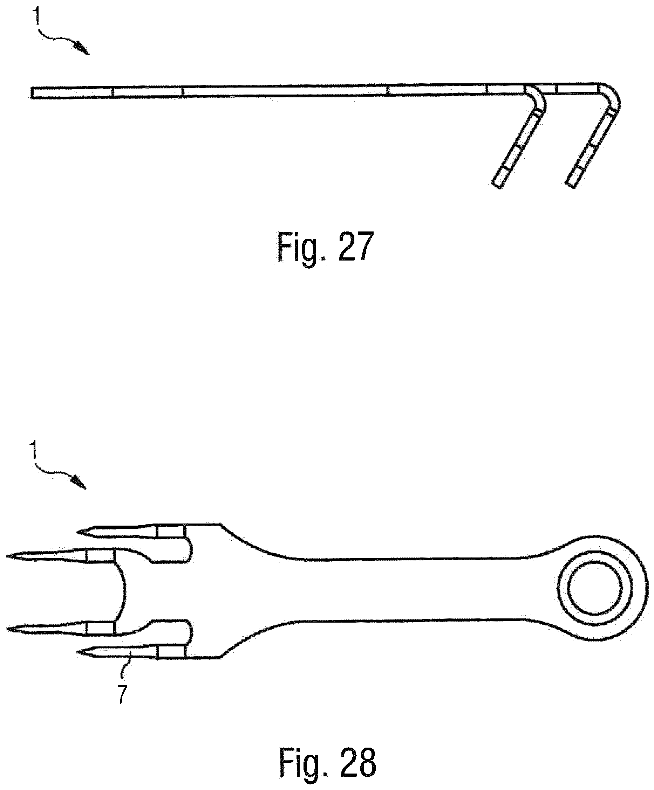

- Fig. 27: shows a side view of the fixation device in Fig. 5;
- Fig. 28: shows a flat top view of the fixation device in Fig. 5;
- Fig. 29: shows a GeoDict "watershed"-graph;
- Fig. 30: shows a GeoDict "porosimetry"-graph;
- Fig. 31: shows the resulting contact area of a surgical fixation device, such as in Fig. 5, with a foam structure;
- Fig. 32: shows a perspective side view of the surgical fixation device in Fig. 24;
- Fig. 33: shows a chirurgical hammer for attaching the surgical fixation device to a foam structure;
- Fig. 34: shows three different surgical fixation devices, all fixed to a foam structure;
- Fig. 35: shows a method for attaching the surgical fixation device (here: hook-plate) to a foam structure (here: metal foam) and the attached combination of both to a bone;
- Fig. 36: shows a foam structure with protruding eyes;
- Fig. 37: shows a foam structure with an alignment indication;
- Fig. 38: shows a further modification of the modification of the foam structure shown in Fig. 14;
- Fig. 39: shows a mandible plate with a foam structure;
- Fig. 40: shows a porous body to be used for spinal interventions;
- Fig. 41: shows a side view (a) and a cross-section (b) of a system with a surgical fixation device provided with an outer thread and one portion of the surgical fixation device comprising a recess filled with a foam structure;
- Fig. 42: shows a femur plate with a foam structure; and
- Fig. 43: shows a cranial implant.

### Detailed Description

### [Fixation Device]

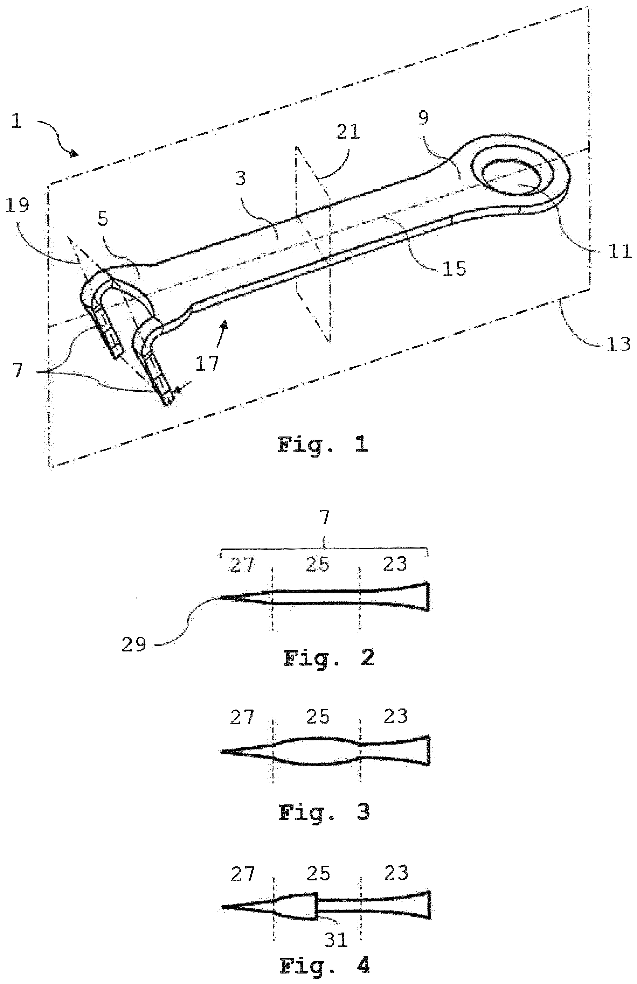

Fig. 1 shows a perspective view of a fixation device 1 for fixation of foam structures, such as open-porous foam structures. Foam structures, in particular implant foam structures, are described below.

The fixation device 1 is shaped as a claw, for example as an elongated hook plate, which provides an elongated plate shaped body 3, which comprises at one side/end 5 thereof two hooks 7 and at another side/end 9 thereof, which is opposite to the one side/end 5, a screw opening 11. The hooks 7 are sized and shaped to be inserted into corresponding porous openings of a foam structure for fixation of the fixation device 1 to the foam structure. The screw opening 11 is sized and shaped to receive a screw for attachment of the fixation device 1 to another solid structure. Such a structure, for example bone, is described below.

The fixation device 1 is symmetric to a first plane 13, in which a main extension direction 15 of the body 3 lies. However, the fixation device 1 may also be asymmetric to the first plane 13, and may be asymmetric to the first plane 13 or any other plane, in which the main extension direction 15 lies.

The hook angle 17 between the body 3 and every one of the hooks 7, measured in parallel to the first plane 13, may be 90° or less, such as anywhere between 45° and 90°. Hook angles 17 within this range contribute to the stability of the fixation between the fixation device 1 and a foam structure, such that the fixation device 1 can absorb both horizontal and vertical forces. Here, the hook angle 17 is 60°.

The hook angle 17 is the same for all hooks 7. However, when there are multiple hooks 7, at least one of the hooks 7 may have a hook angle 17 that differs from the hook angle 17 of at least one of the other hooks 7. Here, the hooks 7 extend in parallel to the first plane 13. However, they may be angled toward or away from the first plane 13 at a plane angle, which may be 90° or less, such as anywhere between 45° and 90°.

Both hooks 7 lie in a second plane 19, also referred to as a hook row, which is orthogonal to the main extension direction 15 and to the first plane 13. However, the second plane 19 may be oblique to the main extension direction 15 and to the first plane 13 such that at least two of the hooks 7 are offset in the main extension direction 15.

Here, the fixation device 1 has hooks 7 only at the one side/end 5. However, it may have hooks 7 at the opposite other side/end 9 as well. In this case, the hooks 7 may face each other and/or may be inclined towards each other. The provision of hooks 7 on both sides 5 and 9 may provide a fixation device 1 that is symmetric to a third plane 21. So, the fixation device 1 may be symmetric to the first plane 13 and/or the third plane 21. Here, it is asymmetric to the third plane 21. Hooks 7 at opposite sides/ends 5 and 9 of the body 3 allow a fixation of two foam structures to each other, of a foam structure and a mesh structure to each other, of a foam structure with a solid structure, or any combination thereof. Examples for such structures are given below. Hooks 7 only at the center of the body 3 are also possible. Thereby, a foam structure can be attached to the center of the body 3, while the opposite sides/ends 5 and 9 of the body 3 comprise attachment structures, such as at least one hook, at least one screw hole, at least one indentation or through-opening. Thereby, one fixation device 1 instead of the two fixation devices 1 used below - for the exemplary fixation of a foam structure to bone - may be used.

Instead of the one screw opening 11 at the other side/end 9 of the body 3, there may be multiple screw openings. Further, there may be not even a single screw opening 11, but any other type of attachment interface. For example, the other side/end 9 may have a specific shape for a form-fit attachment, be made of a specific material suitable for melting, welding etc., or may be suitable for adhesion, for example by means of an indentation or through-opening for receiving adhesive material.

In the fixation device 1, which comprises multiple hooks 7, at least two of the hooks 7 can be of at least one of different length, width, thickness, cross-sectional shape, outer shape, and material in order to achieve various degrees of fixation in different foam structures. Thereby, the fixation device 1 can be securely fixed to a wide variety of foam structures.

Fig. 2 shows the face of the hook 7, when viewed from the lower left side in Fig. 1. The hook 7 has an elongated hook body that is constituted solely by three consecutive sections 23, 25 and 27, which are separated by vertical broken lines in Fig. 2. Each hook 7 has a smaller cross-section than the body 3 from which it extends.

The first section 23 is integral with the body 3. The first section 23 can alternatively be a separate part that is attached to the body 3. The second section 25 follows directly at the end of the first section 23, opposite the end of the first section 23, which is integral with the body 3. The third section 27 follows directly at the end of the second section 25, opposite the end of the second section 25, which is integral with the first section 23. However, the sections 23, 25 and 27 may not be integral. Instead, each section 23, 25 and 27 may form a separate part that is attached to the other part(s) by one of the many known attachment methods in the prior art. Further, each section 23, 25 and 27 may be separated into at least two sub-sections. Theses sub-sections may be modified as already explained and further to be explained with regard to the sections 23, 25 and 27.

The first section 23 is a truncated cone that becomes degressively narrower from its one longitudinal end, which is connected to the body 3, to its other longitudinal end, which is connected to the second section 25. Alternatively, instead of becoming degressively narrower, the first section 27 may become linearly or progressively narrower.

The second section 25 is a cylinder with a uniform cross-section that corresponds to the cross-section at the narrowest end of the first section 23.

The third section 27 is a cone that becomes linearly narrower from its one longitudinal end, which is connected to the second section 25, to its other longitudinal end, which ends at a free tip 29. The free tip 29 has the smallest cross-section along the third section 27 and/or the entire hook 7. Alternatively, instead of becoming linearly narrower, the third section 27 may become degressively or progressively narrower.

Here, the hook 7 is straight. However, it can be arcuate, meandering and/or shaped in a zig-zag-pattern. It is also possible that the hook is helical or in the shape of a corkscrew. The hook 7 is plastically and/or elastically deformable.

Fig. 3 shows a modification of the hook 7 in Fig. 2. The difference being in the second section 25. Instead of a cylinder with a uniform cross-section, the second section 25 has a balloon shape, such that is becomes narrower towards both of its longitudinal ends. Alternatively or additionally, the balloon shape may be provided in the first section 23. The balloon shape may be plastically and/or elastically deformable in a direction orthogonal to the direction of elongation of the hook 7.

Fig. 4 shows another modification of the hook 7 in Fig. 2. The difference being in the second section 25. Instead of a cylinder with a uniform cross-section, the second section 25 has a step 31 that leads to a radially narrowed cross-section along the second section 25 and, thus, to a barb-like configuration. Alternatively or additionally, the step 31 may be provided in the first section 23, the third section 27, between the first section 23 and the second section 25, and/or between the second section 25 and the third section 27. The step 31 may be plastically and/or elastically deformable in a direction orthogonal to the direction of elongation of the hook 7.

The taper along the hook 7 in Fig. 3 and Fig. 4 created by the balloon shape or the step 31 serves the purpose of interlocking the hook 7 with pores of a foam structure.

The size, shape and/or material of the hooks 7 may be adapted to allow a plastic deformation caused by the intended foam structure, to which the fixation device 1 is to be attached.

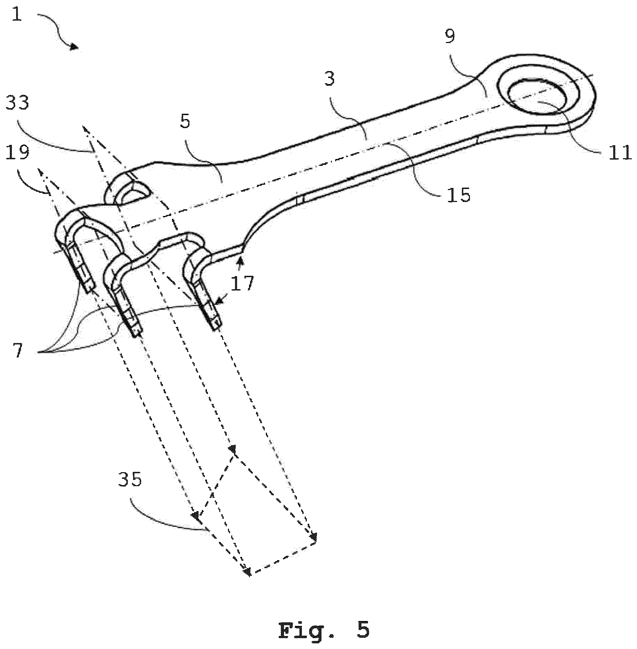

Fig. 5 shows a perspective view of a modification of the fixation device 1 in Fig. 1. The difference being in the one side/end 5, where instead of two there are now four hooks 7, three of which are visible in the figure. In addition to the two hooks 7 in the second plane 19, there are now two more hooks 7 in a fourth plane 33, which is parallel to the second plane 19, such that all hooks 7 extend in parallel to each other. Thereby the hooks 7 are offset in the main extension direction 15 and form corners of a trapezoid area 35, i.e. they span a trapezoid area 35, which is available for fixation to a foam structure. The trapezoid area 35 is parallel to the main extension direction 15. In the figure, the trapezoid area 35 is shown as a lower projection of the actual location of the area in order to avoid confusion with the second plane 19 and the fourth plane 33.

In fact, the second plane 19 and the fourth plane 33 may be referred to as hook rows 19 and 33, where the second plane 19 is the first hook row 19, comprising hooks 7 that are farthest away from the other side/end 9, and the fourth plane 33 is the second hook row 33, comprising hooks 7 that are closer to the other side/end 9 than the hooks 7 of the first hook row 19. There may be one hook row (see the fixation device 1 in Fig. 1) or more than two hook rows, such as three, four, five or six. Further, each hook row may comprise one or more than one, i.e. multiple, hooks 7, such as two, three, four, five or six. A hook row, such as the aforementioned second plane 19 and fourth plane 33, is defined by the extension of one or more hooks 7 in a plane which is orthogonal to the main extension direction 15.

The hooks 7 of the first hook row 19 are as long as the hooks 7 of the second hook row 33. However, at least one hook 7 of one hook row may differ in length compared to another hook 7 of the same one hook row and/or compared to yet another hook 7 of a different hook row. Hooks 7 of one hook row may have equal lengths. Hooks 7 of the one hook row may have different lengths than hooks 7 from another hook row. For example, the hooks 7 of the first hook row 19 may be shorter than the hooks 7 of the second hook row 33. Alternatively, the hooks 7 of the first hook row 19 may be longer than the hooks 7 of the second hook row 33.

The hooks 7 of the first hook row 19 have the same hook angle 17 as the hooks 7 of the second hook row 33. However, at least one hook 7 of one hook row may differ in hook angle 17 compared to another hook 7 of the same one hook row and/or compared to yet another hook 7 of a different hook row. Hooks 7 of one hook row may have the same hook angle 17. Hooks 7 of the one hook row may have different hook angles 17 than hooks 7 from another hook row. For example, the hooks 7 of the first hook row 19 may have a smaller hook angle 17 than the hooks 7 of the second hook row 33. Alternatively, the hooks 7 of the first hook row 19 may have a larger hook angle 17 than the hooks 7 of the second hook row 33.

The hooks 7 of the first hook row 19 have a smaller distance to each other compared to the hooks 7 of the second hook row 33. However, hooks 7 of at least one hook row, such as the first hook row 19, may have the same distance to each other as hooks 7 of another hook row, such as the second hook row 33. Hooks 7 of one hook row, such as a hook row with three or more hooks 7, may have different distances to each other. The hooks 7 may be arranged symmetrically in their hook row. Hooks 7 of one hook row may be offset in the main extension direction 15 to hooks 7 of an adjacent hook row, as shown here.

### [Foam Structure]

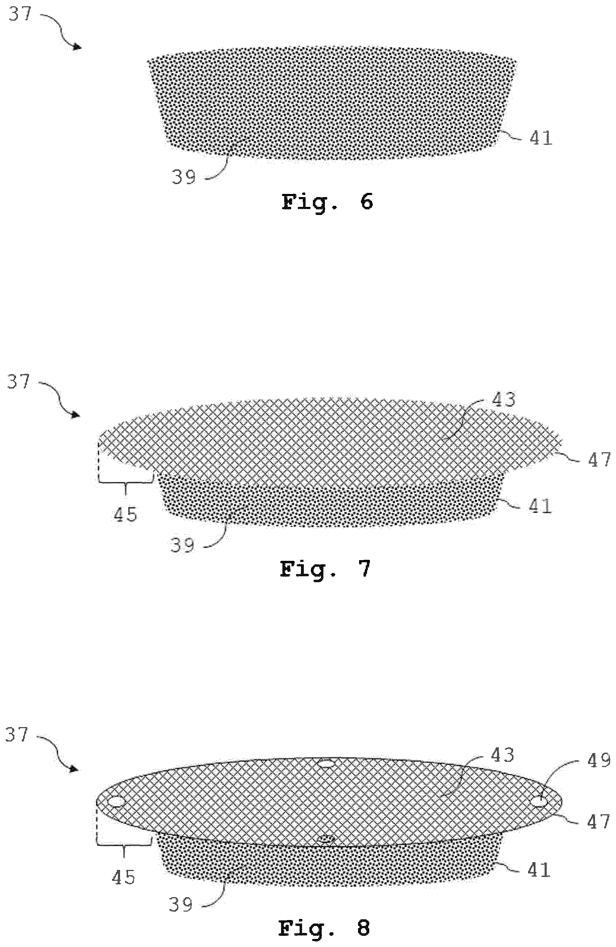

Fig. 6 shows a perspective view of a foam structure 37 consisting of a porous body 39. In this disclosure, the foam structure 37 is an implant for craniofacial use (e.g., for closing a bone defect in a patient's skull). However, it can be used in other anatomic regions, in particular in the CMF region, as well.

In this disclosure, it is understood that the term foam refers to an open-porous solid structure, which at least partially surrounds at least one of a gaseous material, liquid material and solid material.

The foam structure 37 can be a 3D-printed metallic (e.g., titanium) foam. The foam may have a coating, which comprises or consists of hydroxyapatite (HA) and which has a thickness of, for example, 20 µm. The porous body 39 is a truncated cone with an outer top surface, an outer bottom surface, which is smaller than the top surface, and an outer side surface, which connects the top and bottom surfaces. These surfaces form an outer body edge 41, which is open to all sides. The resulting angled lateral/side body edge 41 between the top surface and the bottom surface provides, in bone for example, an improved distribution of force - from the outside to the inside of the body - to the bone. So, any porous body 39 that is not a truncated cone may be modified by adopting the shape of a truncated cone, wherein the angled lateral/side body edge 41 is the important aspect here.

In some variants, the foam structure 37 may not be 3D-printed but manufactured by a different conventional method, such as the "Schwarzwalder method." The material used may not be titanium (Ti) but another material, such as another metal (e.g., stainless steel) and/or polymer material. The foam structure 37 may not be made of titanium (Ti) but comprise or consist of one or multiple other biocompatible implant materials, such as resorbable biocompatible implant materials.

The foam structure 37 is monolithic and made from the same material. However, the foam structure 37 may be manufactured by connecting/attaching several parts made of the same or different materials. The connecting/attachment means/method for this purpose may be any means/method known in the prior art.

The porosity of the foam structure 37 is uniform. However, the porosity of the foam structure 37 may vary. For example, it may follow a specific pattern. The porosity may vary, such as decrease or increase, from the body edge 41 towards the center of the foam structure 37.

The foam structure 37 comprises a hydroxyapatite (HA) coating, which is either fully or partially provided on the foam structure 37. This includes the application of the coating throughout the foam structure 37, i.e., through the foam structure 37, such that it reaches inner pores of the foam structure 37 as well. The thickness of the coating may be within the range of 15 to 25 µm. Additionally or alternatively, the coating may be such that particles of the coating material, e.g. hydroxyapatite (HA), cannot detach from the coating on the foam structure 37, when the foam structure 37 is bend, for example during use of the foam structure 37. Hydroxyapatite (HA) stimulates bony ingrowth. Consequently, other coating materials may be used, which are capable of stimulating bony ingrowth.

The foam structure 37 is not limited to being shaped as a truncated cone. In fact, it may have any shape deemed suitable for the intended use, such as a shape with a uniform cross-section, for example a cylindrical shape.

Here, the foam structure 37 consists of the porous body 39. However, the foam structure 37 may comprise the porous body 39 along with other components. A few such examples are described with the modifications below.

### [System from foam structure and monolithic or non-monolithic fixation device]

Fig. 7 shows a modification of the foam structure 37 in Fig. 6. Here, an attachment structure 43, e.g., another surgical fixation device such as a mesh-type structure or any other stabilizing structure, is provided at the top of the porous body 39. The attachment structure 43 in same variants is monolithic and made from the same material as the porous body 39. In fact, the attachment structure 43 and the porous body 39, which constitute the entire foam structure 37, may be 3D-printed as one piece, i.e., as one monolithic structure. Alternatively, the attachment structure 43 may be made as a separate piece and/or may be made of a different material than that of the porous body 39. Even when the attachment structure 43 and the porous body 39 are separate pieces, they still may both be 3D-printed and may be fixed to each other, for example by a welding, press-fit, undercut, screwing, hooks and/or the fixation device according to this disclosure. In particular, cold welding techniques may be used to fix the attachment structure to the porous body.

The attachment structure 43 is shaped as a plate/sheet and comprises an edge portion 45, i.e., a first attachment structure, which extends from the body edge 41 of the porous body 39. The attachment structure 43 is entirely made of a mesh structure and has a mesh edge 47 around the edge portion 45. Here, the mesh edge 47 is open. The mesh structure may be elastically and/or plastically deformable. Being plastically deformable allows the mesh structure to be adapted to a patient's natural bone surface geometry, for example.

The mesh structure comprises holes (i.e., mesh openings), which at least in the edge portion 45 are adapted to receive screws. These holes may vary in size, such that differently sized screws can be used.

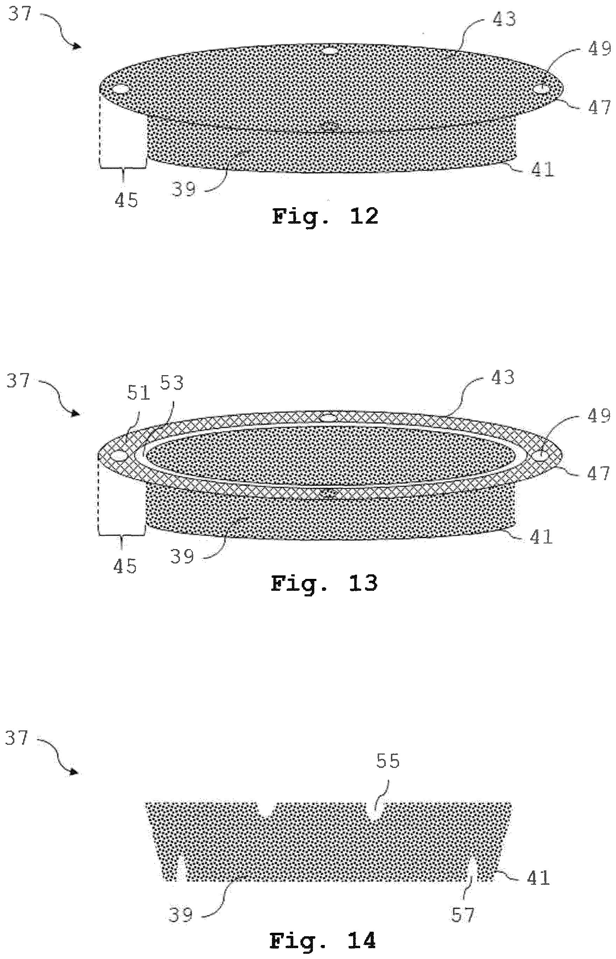

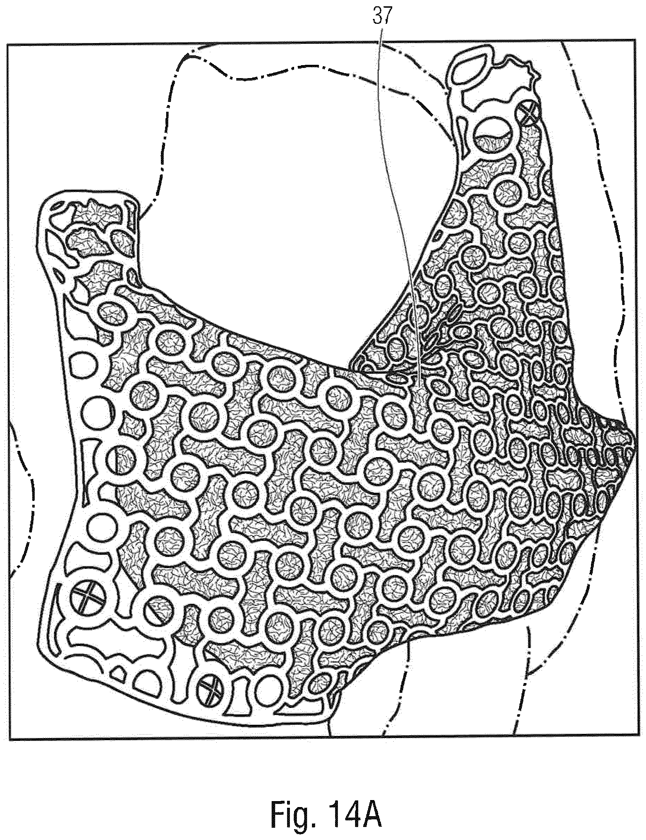

Fig. 8 shows a modification of the foam structure 37 in Fig. 7. Again, the mesh-type fixation device 43 and the foam structure may be provided as a monolithic structure or as dedicated (non-monolithic) components that are manufactured separately and later on attached to one another. Figs. 14A and 1B illustrate use cases for the (e.g., patient-specific) monolithic variant of Fig. 8 for implantation in the orbita region (eye socket) and the cranial region, respectively. The mesh has been selected to have a configuration as disclosed in EP 0 654 250 A1 and may monolithically be manufactured with the porous body 39 using, for example, 3D printing.

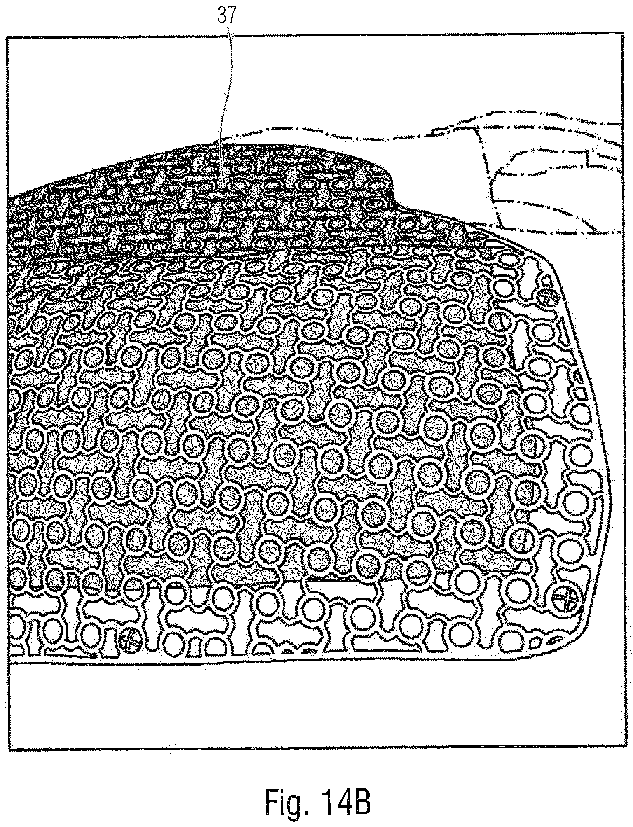

In Fig. 8, the mesh edge 47 is a fully closed bar/frame. Further, a number of attachment openings 49 through the edge portion 45 are distributed at (e.g., equal) circumferential distances on the edge portion 45 and around the porous body 39. Each attachment opening 49 is sized and shaped to receive a bone screw for attachment of the foam structure 37 to a solid structure, such as bone (see also Figs. 14A and 14B showing a few exemplary screws). Each attachment opening 49 may differ in shape and/or size to its adjacent holes of the mesh structure. Instead of four as schematically shown in Fig. 8, the attachment openings 49 may be at least one or more, such as two, three, five or six, depending on the size of the foam structure 37 and the characteristics of the solid structure, to which it shall be attached. The provision of dedicated circular attachment openings 49 may be omitted, when the mesh structure at least in the edge portion 45 comprises mesh openings, which are adapted to receive screws. Further, the mesh edge 47 may remain open, as in Fig. 7.

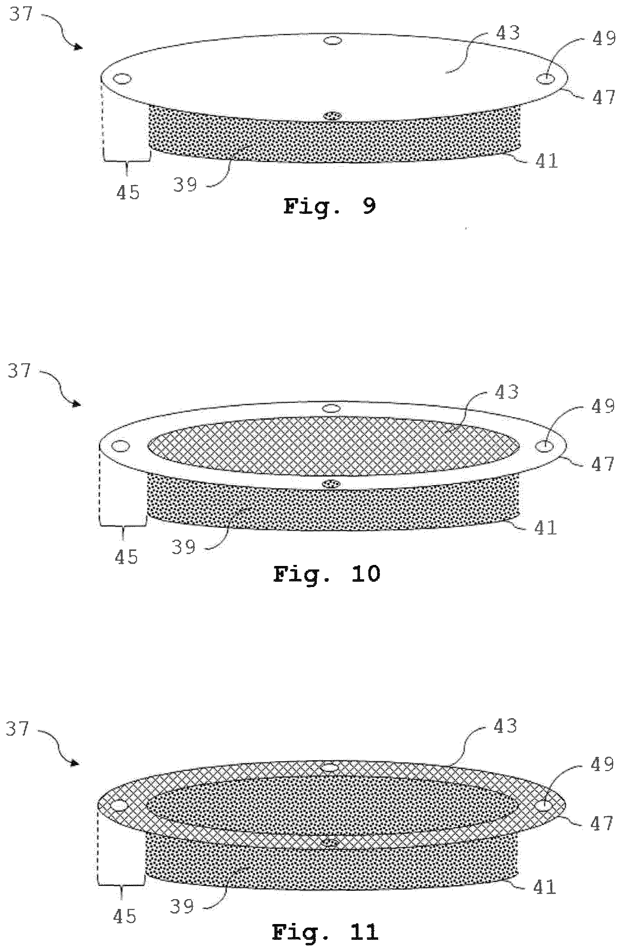

Fig. 9 shows a modification of the foam structure 37 in Fig. 8. Here, the attachment structure 43 is made of a solid titanium (Ti) plate/sheet. While the attachment structure 43 may be circular, oval or ring shaped, it may just as well have any other shape or form. Further the body edge 41, i.e., the bottom side of the porous body 39, opposite the attachment structure 43 and opposite the top side of the porous body 39 is a fully closed layer at the outer bottom side of the porous body 39. The layer may be a partially closed layer. When used as an implant with this fully closed layer being proximal to the patient, it prevents ingrowth of dura mater and/or contact of dura mater to high concentration of calcium (Ca). Also, the porous body 39 is not shaped as a truncated cone but has a uniform cross-section, in particular a cylindrical shape.

Fig. 10 shows a modification of the foam structure 37 in Fig. 9. Here, only the edge portion 45 is made of a solid titanium (Ti) plate/sheet.

Fig. 11 shows a modification of the foam structure 37 in Fig. 10. Here, the attachment structure 43 consists only of the edge portion 45 and is entirely made of a mesh structure.

Fig. 12 shows a modification of the foam structure 37 in Fig. 11. Here, the edge portion 45 is entirely made of the foam structure of the porous body 39.

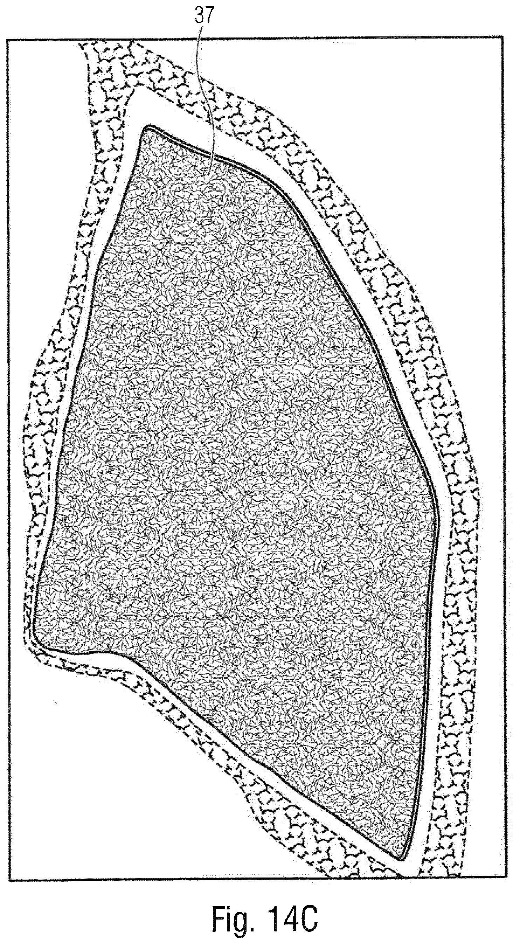

Fig. 13 shows another modification of the foam structure 37 in Fig. 11. Here, the edge portion 45 is formed by an outer section 51 of a mesh structure (as explained above) and an inner section 53 (i.e., rim or edge) of a fully closed configuration, which is directly attached to the outer section 51, see also Fig. 14C (which shows a patient-specific implant suitable for closing an opening in the cranial region). The inner section 53 may extend further onto or into the porous body 39.

Fig. 14 shows a side view of another modification of the foam structure in Fig. 6. Here, the foam structure 37 comprises top recesses 55, which allow the reuse of existing bone chip pieces, and bottom recesses 57, which improve bendability of the foam structure 37. Of course, in certain variants only top recesses 55 or only bottom recesses 57 may be provided. Such recesses 55, 57 may be provided in any of the foam structure embodiments discussed above. Further, recesses with a small offset, i.e., one to several millimeter, compared to a bone cutting line from the top of the foam structure 37 may be provided, for example in the form of a spider web, to allow pushing the foam structure 37 to a bone, if the foam structure 37 does not perfectly fit the bone. Of course, the aforementioned modification(s) can be applied to any foam structure 37 with an attachment structure 43 and/or with an edge portion 45.

In this disclosure, the edge portion 45 may be fully or partially around the foam structure 39.

In this disclosure, the attachment structure 43, the edge portion 45, its outer section 51 and/or its inner section 53, and/or the mesh edge 47 may each be made of titanium (Ti). However, they may also not be made of titanium (Ti) but of a titanium alloy, comprise or consist of one or multiple other biocompatible implant materials, such as resorbable biocompatible implant materials.

In this disclosure, the foam structure 37 may allow drilling of holes into it and/or through it.

Further, in this disclosure, the foam structure 37 may comprise a printed or laser engraved marking, indicating an anatomic orientation and/or identifying the patient, such as an anterior orientation of a respective part of the foam structure.

The foam structure 37 may be adapted to be sufficiently stable to assure safety against pressure from outside and inside a patient's body, to allow slight bending to assure a good fit to or in bone, and/or to allow being cut by a mechanical or handheld electronic tool typically used by a surgeon.

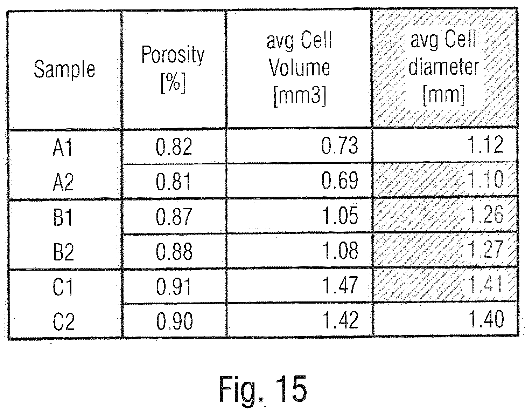

Fig. 15 shows a table of samples with different foam characteristics. The samples being denoted as A1, A2, B1, B2 and C1, C2. The measurements illustrated in Fig. 15 have been made before the coating process.

### [Further System and Method]

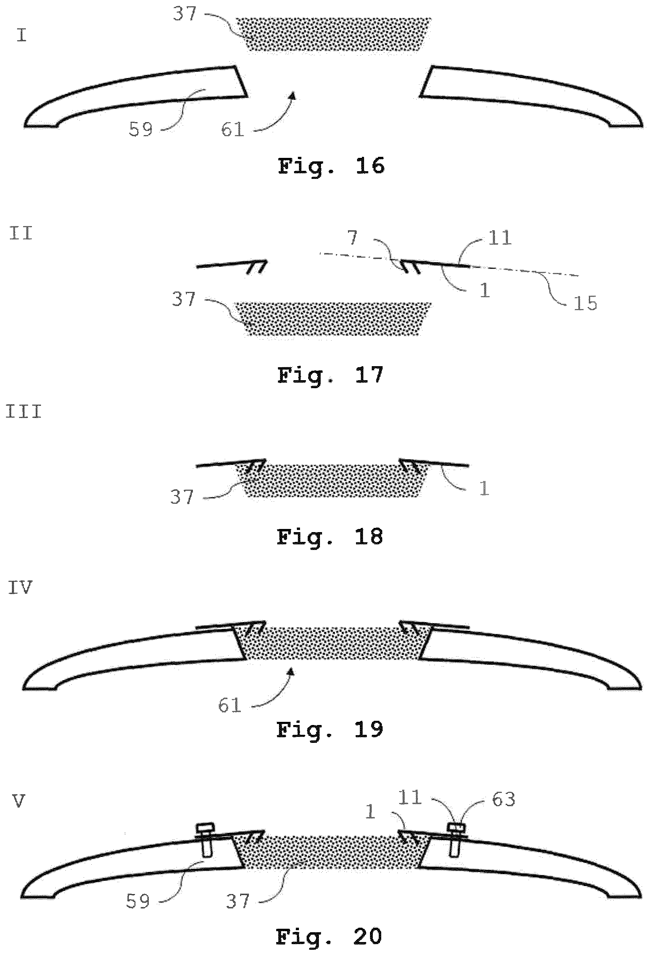

Fig. 16 shows a side cross-sectional view of a portion of a craniofacial bone structure 59 with an opening 61 to be closed by any one of the aforementioned foam structures 37, including the respective modifications of the foam structures 37. The craniofacial bone structure 59 is used for illustrative purposes. However, it may be any other bone structure.

Fig. 16 also illustrates a first step I of either providing a foam structure 37 with the dimensions of the opening 61 or creating an opening 61 with the dimensions of a foam structure 37. In other words, the foam structure 37 may be manufactured, e.g. 3D-printed, to fit specific dimensions of the opening 61. An alternative is the provision of a series of differently shaped and sized foam structures 37, of which one is chosen by the surgeon such that it resembles the already existing opening 61 or an opening that is yet to be created or was already created during surgery. In this case, the foam structure 37 may be adapted to allow slight plastic and/or elastic bending to assure a good fit, and/or to allow being cut by a mechanical or handheld electronic tool typically used by a surgeon to assure a good fit. The foam structure 37 may be as wide as the opening 61 or narrower than the opening 61. Width is measured from wall to wall of the opening 61 (left-right-direction in Fig. 16). The foam structure 37 may further be as thick as or thinner than the adjacent craniofacial bone. Thickness is measured orthogonally to the width (up-down-direction in Fig. 16).

Fig. 17 shows a system, comprising the foam structure 37 in Fig. 16 and the fixation device 1 of, e.g., Fig. 5.

Fig. 17 also illustrates a second step II of choosing the fixation device 1 that is suitable for the size, porosity and material of the foam structure 37. For this purpose, the cross-section of the hook 7 may correspond to or be less than the avg pore size, i.e. the avg Cell diameter, of the foam structure 37. Additionally or alternatively, the mechanical strength of the hook 7 may be provided such that a plastic deformation of the hook 7 occurs before a plastic deformation of the foam structure 37, when the fixation device 1 is being fixed to the foam structure 37, in order to minimize destruction of the foam structure 37. Thereby, the hook 7 can interlock into the foam structure 37 and a proper fixation of the fixation device 1 to the foam structure 37 can be achieved, while preserving the functionality of the foam structure 37. Further additionally or alternatively, the hook angle 17 can be provided such that it corresponds to the general pore arrangement in the foam structure 37. It should further be mentioned that the fixation device 1 may comprise at least three hooks 7 with an offset arrangement, i.e. of which at least two are offset in the main extension direction 15. This creates an area, such as the trapezoid area 35 created by the four hooks 7 in Fig. 5, in order to enhance the stability of the fixation between the fixations device 1 and the foam structure 37.

Fig. 18 shows the system in Fig. 17 in a state, where the fixation device 1 is fixed to the foam structure 37.

Fig. 18 also illustrates a third step III of attaching/fixing the fixation device 1 to the foam structure 37, for example by a surgical hammer. This allows for a simple and time-saving handling.

Fig. 19 shows the system in Fig. 18 in a state, where the system is inserted into the opening 61.

Fig. 19 also illustrates a fourth step IV of inserting the system into the opening 61. Prior to inserting, the orientation of the system in accordance with the opening 61 and its surrounding solid structure, here bone, may be adjusted.

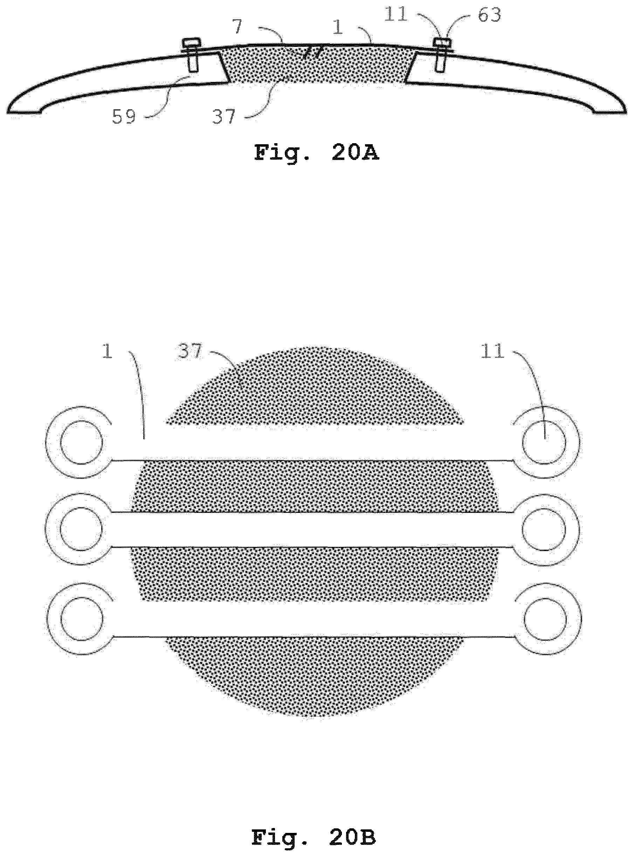

Fig. 20 shows the inserted system in Fig. 19 in a state, where the system is attached/fixed to the craniofacial bone structure 59 with a screw 63 passing through the screw opening 11 of the fixation device 1.

Fig. 20 also illustrates a fifth step V of attaching/fixing the system to the craniofacial bone structure 59 with a screw 63 passing through the screw opening 11 of the fixation device 1.

The system may comprise any one of the aforementioned foam structures 37, including the respective modifications of the foam structures 37, and a cranial plate, instead or in addition to the aforementioned fixation device 1, including the respective modifications of the fixation device 1.

Fig. 20A shows a modification of the system in Fig. 20. The difference being the provision of a fixation device 1, i.e., a stabilizing structure, which extends laterally over the foam structure 37. In this case, the fixation device 1 comprises a screw hole/screw opening 11 at each end and hooks 7 at its central region. Of course, the screw hole/screw opening 11 may be referred to a bone attachment region capable of being attached to bone, for example by any other means/method known in the prior art.

Fig. 20B shows a top view of the modification in Fig. 20A. In this exemplary illustration, three fixation devices 1 are used. However, their number may vary. The three fixation devices 1 constitute geometric entities, which may have a solid body (i.e., of a line type) or body with a mesh structure, which also applies to the body 3 of any other fixation device 1 of the disclosure.

The fixation device 1, i.e., the stabilizing structure, may be monolithically formed on one side, the top side in Figs. 20A and 20B, of the foam structure and may be monolithic with the foam structure.

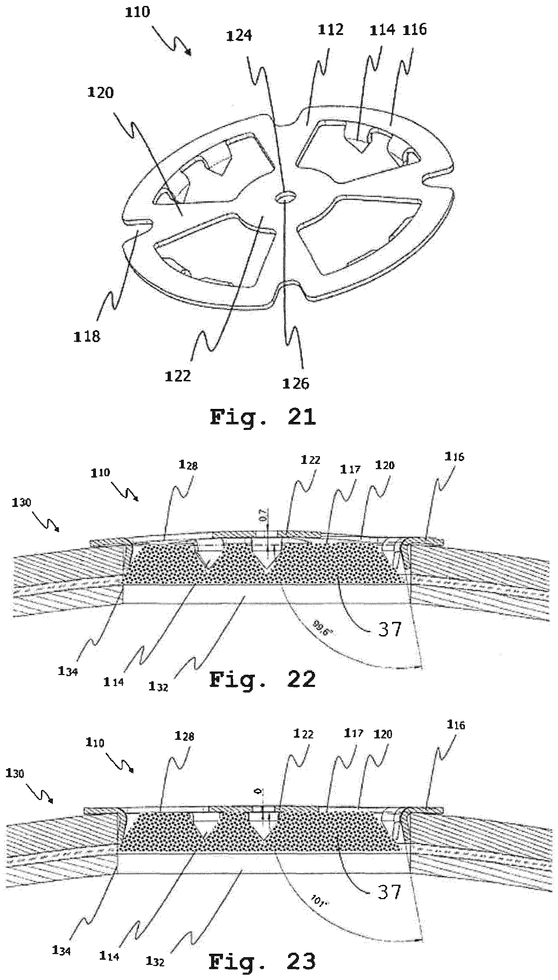

Fig. 21 shows a cranial plate 110 similar to the one known from EP 3 441 030 A1. The cranial plate 110 comprises a plate body 112; and a plurality of spikes 114 extending from the plate body 112, the spikes 114 configured to engage bone, such as cranial bone. Alternatively, the cranial plate 110 comprises: a plate body 112; and a plurality of spikes 114 extending from the plate body 112, the spikes 114 configured to engage bone, such as cranial bone, wherein the plate 110 is deformable to enable movement of the spikes 114 from a disengaging configuration to an engaging configuration upon deformation of the plate 110. Alternatively, the cranial plate 110 comprises a plate body 112; and a plurality of spikes 114 extending from the plate body 112; wherein the plate body 112 has a burr hole covering portion configured to assume one of a convex form with a positive curvature, a flat form with zero curvature and a concave form with a negative curvature when the spikes 114 are in a disengaging configuration; wherein the spikes 114 are configured to engage bone, such as cranial bone, wherein the plate 110 is deformable to enable movement of the spikes 114 from the disengaging configuration to an engaging configuration upon deformation of the plate 110, wherein the plate body 112 is deformable so that the plate body 112 assumes a form with a decreased curvature when the spikes 114 are in the engaging configuration, wherein a decreased curvature comprises deforming the plate body form from a convex form to a flat form, a convex form to a concave form, a flat form to a concave form, a convex form to a less convex form or a concave form to a more concave form.

Fig. 22 shows the disengaging configuration associated with the cranial plate 110 in Fig. 21.

Fig. 23 shows the engaging configuration associated with the cranial plate 110 in Fig. 21.

Figs. 21 to 23 correspond to the original Figs. 1A, 2 and 3 of EP 3 441 030 A1, wherein in front of each reference sign in the original figures a "1" is placed in order to avoid confusion with the reference signs already used in the other figures of this disclosure. Further, in Figs. 22 and 23 a foam structure 37 has been added to illustrate a state, in which the cranial plate 110 and the foam structure 37 form a system.

In combination with the cranial plate 110 of EP 3 441 030 A1, the foam structure 37 may be arranged on (e.g., attached to) the side of the plate body 112, from which the spikes 114 extend, such that the spikes 114 radially enclose the foam structure 37, see Figs. 22 and 23. This system may be adapted such that the foam structure 37 does not impede with the deformation movement of the cranial plate but assures the contact of the foam structure 37 to the bone. For this purpose, the cranial plate 110 may replace the attachment structure 43 described above, including all of its modifications. It may replace the fixation device 1 as well. Further, the cranial plate 110 may comprise predetermined breaking points 114a to easily detach after some time in the body. The breaking points 114a may be provided on the spikes 114, preferably below their attachment on the plate body 112. Thereby, the surgeon can easily perform the detachment and the remaining structure in the body is left with a "zero-hight profile", i.e. no longer protruding from the surrounding bone.

### [Further Aspects]

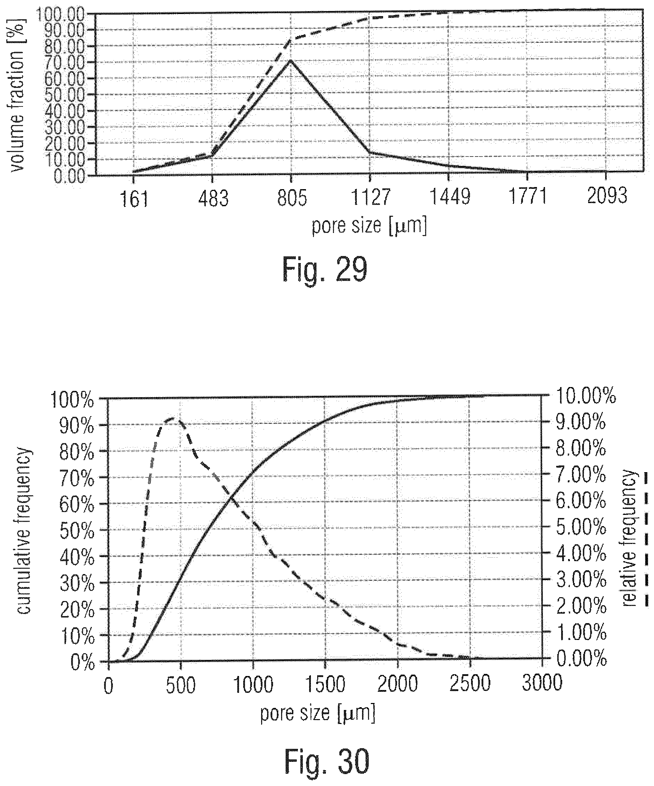

Further, aspects of the present disclosure, in particular with respect to the above embodiments, relate to:
[1]
   [a] A foam structure (e.g., Ti foam) and/or
   [b] fully or partially hydroxyapatite-coated with for instance a thickness of 20µm +/-5µm, so that particles of Hydroxyapatite cannot result out of potential bending of the device.
      [a] may further:
         [i] be 3D-printed together as one part with a plastically deformable, overlapping mesh on top of the foam, wherein, as an option,
            [1] this mesh is surrounded by a closed bar / frame, and/or
            [2] this mesh includes screw holes for fixation of the implant to the mesh, which is adapted to patient's natural bone surface geometry; and/or
         [ii] be statistically defined pores in a certain range (e.g., of ca. 400 to 1200 µm, in particular ca. 500 to 1000 µm).
            Defined by "watershed"
               - see Fig. 29, where:
                  Porengröße = size of pores, Vol-Anteil = percent by volume, continuous curve - relative frequency, dashed curve - cumulative frequency
            Defined by "porosimetry"
               - see Fig. 30, where:
                  Porengröße = size of pores, Vol-Anteil = percent by volume, dashed curve - relative frequency, continuous curve - cumulative frequency.
            The measurements can be performed and/or analyzed using GeoDict by Fraunhofer Institute.
            Further information on foam and pore characteristics like the analyses of pore dimensions can be found in Fig. 15; and/or
         [iii] have a height according to the patient's natural bone thickness (the height of the implant can also be slightly smaller than the natural bone thickness of the patient to avoid pressure if fit is not 100% accurate to the brain).
[2] The aforementioned, wherein thickness and/or bar width and/or pattern of mesh, and/or thickness and/or bar width and/or porosity of foam being:
   [a] sufficiently stable to assure safety against pressure from outside and inside; and/or
   [b] allows slight bending of complete implant to assure a good fit to the cut bone; and/or
   [c] enables the surgeon to cut the implant with mechanical or handheld electronic tools.
[3] The aforementioned that allows drilling of holes into the foam structure.
[4] The aforementioned with a printed marking on the implant (e.g., for easier anatomical orientation and/or indication of patient identifier).
[5] The aforementioned with orthogonal edge or with angled edge (so that force can be easily transmitted to the bone) to tangential plain of skull bone surface and instruments to create such an angled edge (bone at proximal end has a smaller diameter than at distal end) (complete implant as shown in [1] or [2] is dedicated for craniofacial use but can be utilized in other areas too).
[6] The aforementioned but mesh and foam not out of Ti but out of one or multiple biocompatible implant materials, also resorbable materials; combinations of different materials are also possible.
[7] The aforementioned but mesh and foam are printed separately and then fixed by mechanical means together or by additional process steps (e.g. welding, press fit, undercut, screwing, hooks).
[8] The aforementioned but with other production methods than 3D-printing (e.g. Schwarzwalder method and welding it together with mesh).
[9] The aforementioned but
   - without surrounding bar of mesh, and/or
   - without overlapping mesh adapted to patient's natural bone surface geometry, and/or
   - without overlapping mesh, and/or
   - without mesh in total, and/or
   - with alternative fixation method to bone - deformable hooks in Ti plate (see EP 3 441 030 A1), and/or
   - implant with or without mesh is adapted by the surgeon to the patient specific geometry.
[10] The aforementioned but with mesh being replaced by a solid Ti layer with screw holes.
[11] The aforementioned but with partially or fully closed proximal / lower layer to avoid ingrowth of Dura mater or contact of Dura mater to high concentration of Ca.
[12] The aforementioned but with mesh printed fully or partially only around foam with transition zone from mesh to foam with cells completely out of Ti.
[13] The aforementioned but with recesses in foam to allow reuse of existing bone chip pieces of the patient.
[14] The aforementioned but with small recesses in foam:
   [a] from below of the implant to allow a better bendability of foam, and/or
   [b] with a small offset compared to the bone cutting line (in the order of one to several millimeter) from the top of the implant and e.g. in the form of a spider web to allow pushing the foam to the bone if during cutting a/the form of the implant could not be met.
[15] A combination of the foam with EP3441030A1 and/or EP3441030B1 so that below the implant mentioned the aforementioned foam can be found - not impeding with the deformation movement of the implant but assuring the contact of the foam to the bone.

### [An exemplary medical use case being addressed by the present disclosure]

Craniofacial implants do typically not show bone ingrowth, except if ceramic materials are used but they have important disadvantages like brittle fracture. With bone ingrowth (German: "Einwachsen") a better mechanical stability is expected. Additionally, bony ingrowth has been limited with other implants based on bone cements to ongrowth (or max. depth 0.2mm, German: "Anwachsen"). For critical sized defects (body of patient is not able to close it on its own) bone ingrowth can be achieved with the present disclosure shown in this / the corresponding documents. Furthermore, with the solution a reduction of infection rates is expected since the immune system is able to get to all sites of the bony void filled with the implant.

Titanium as one of the materials used in this disclosure is highly biocompatible and the experience with this material is important.

Defects in the craniofacial bone structure can have different causes. These include, for example, intracranial tumors, craniocerebral traumata or vascular malformations. In these cases, the bone defects can be reconstructed with implants. The ideal in the reconstruction of bone defects is the achievement of the condition "restitutio ad integrum". This refers to the restoration of the functional state of the bone tissue that existed before the disease. The importance besides others is especially given for so called critical size defects. Implants with a foam-like/porous structure may enable bony ingrowth into the implant structure. The advantage of using a porous implant structure instead of a solid structure is to achieve possible bone ingrowth. Foam-like implants can be used not only in the CMF area but also in the area of spine surgery. Implants with foam structures on a rigid implant are already successfully used in knee and hip surgery. Titanium foam is a possible implant material to achieve bone ingrowth and to provide a basic stability.

These implants must be rigidly fixed to the bone to provide sufficient stability. This stability is needed to improve possible bone ingrowth and to protect the underlying tissue from external forces. Usually implants in the CMF area can be fixed with plates and screws. These screws are usually designed to be inserted into solid structures respectively structures which can take up high loads in small areas. However, this type of Benchmark fixation might have an insufficient stability when used on porous materials. This novel surgical fixation device is specifically designed for fixation of implants with a foam-like structure. It is understood that the word foam refers to an open-porous solid material at least partially surrounding another gaseous, liquid or solid material.

Foam-like structures are not only used in medical technology. Also, in the aerospace, gas / oil drilling, automotive, construction industry or other industrial sectors foam-like structures must be attached. The novel product could also be used in these industries for fixing foam-like parts. The described product shall offer a stable, simple, fast and effective device for foam fixation.

### [Technical solution to the use case that currently exists]

As mentioned above ceramic implants exist with the corresponding disadvantages. Additionally, Titanium open porous implants are known but differing in important features like pore sizes, no coating etc.

Currently the technical solution for fixation of implants in the CMF area are plates & screws, suture and clamp-systems. These fixation systems are not specifically designed for the application on porous materials. So far porous (metal) implants have not been used in the CMF area. Therefore, there is no specific technical solution for fixing such implants that currently exists. The disadvantages are the missing stability or the need to use more than one surface of the object to be fixated.

Foam structures as mentioned above are also used in other industries. A proper fixation of the components in these industries is a very important aspect. Therefore, the development of a foam-specific technical solution for fixation of foam-like components could also be used in these industries.

The herein described product shows a novel fixation device which is explicitly designed for the fixation of foam-like implants/components.

### [How aspects of the disclosure work]

One idea is to create a fixation device which is specifically designed for the fixation of foam-like structures. The product shall offer an effective way to fixate the foam properly and at the same time preserve the functionality of the foam structure. Therefore, the product, i.e., a hook plate as generally presented herein, is developed. The hooks are located at one side of a plate which can be inserted into ideally existing openings of the foam to reach a proper fixation. On the other side of the plate a screw can be inserted to attach the porous implant to the bone or another solid structure or other techniques can be used for fixation on this side.

The dimensions of the hooks are specifically designed to fit into the foam structure (maximize stability and the potential deformation of the hook and minimization of any damages by maximizing the ease of insertion). For the (mechanical) design of the hook plate various aspects are taken into account.

For example, the hooks should follow the path of the lowest resistance into the foam- structure. The deeper they are inserted the more difficult it should be to retrieve them. For this product, the hook size is aligned to the pore size of the foam. Additionally, the mechanical strength of each hook is considered. On the other hand, particle formation by destroying the foam when inserting the hooks should be minimized. In this aspect, the pore size of the foam serves as a limiting factor. In addition, the plastic deformation of the hook should occur before the deformation of the foam starts. With this specific design the hooks can interlock themselves into the foam structure and at the same time minimize damage of the porous material. The hook should always be inserted in more than one pore of the foam.

The resulting stability of the fixation arises from two different design aspects. The interlocking system of the hooks provides an important part to reach a proper stability of the fixation device if the implant is exposed to a corresponding bending load. Additionally, designing the hooks with an offset arrangement can increase the resulting contact area and therefore enhance fixation stability.

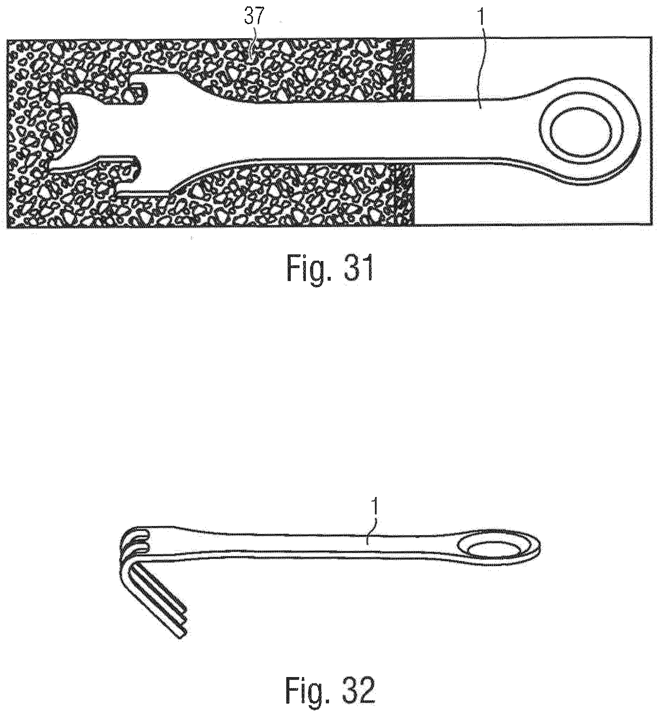

Fig. 31 shows the resulting contact area of the surgical fixation device with a foam structure.

The angle between the hooks and the plate contributes to additional stability of the fixation. Therefore, the angle between the plate and the hooks preferably is between 45° and 90°. With this specific angle the novel fixation device can absorb both horizontal and vertical forces. When choosing the optimal angle, in addition to the resulting stability the usability must be considered to provide an easy application of the fixation device.

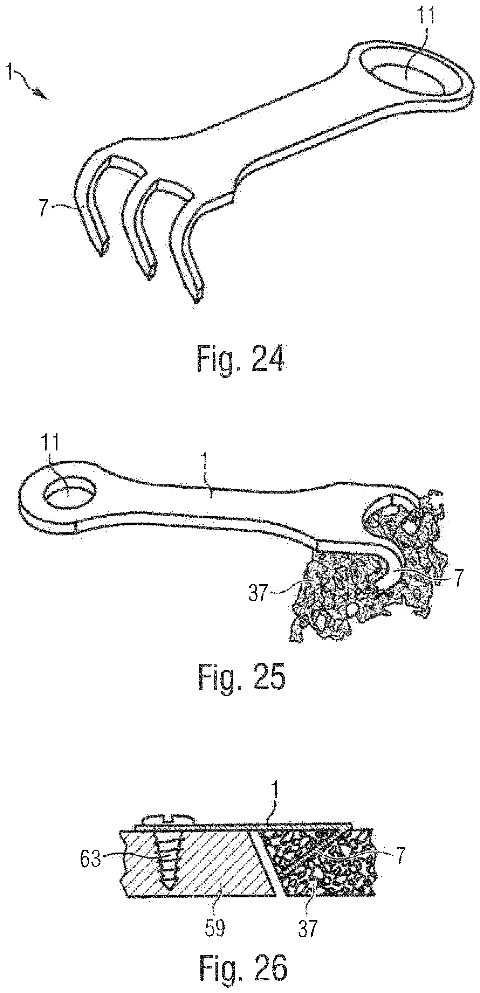

Fig. 32 shows a perspective side view of the surgical fixation device in Fig. 24, wherein the angle between the body and the hooks is between 45 and 90° considering stability and usability.

The stability of the hook-plate fixation is evaluated with a biomechanical load test. For this purpose, the Lerch-test is performed. Within this test a force is applied to the implant and the fixation simulating the mechanical situation at the cranial region. The same test is also used for the Universal Neuro 3 implant system already on the market since a few years. The results show sufficient stability of the novel hook plate for the fixation of cranial implants. Also, for the fixation of implants on the midface, the novel fixation device exhibits sufficient stability.

The usability offers an improved, i.e. simple and timesaving, handling compared to the use of plates and screws. The hook plate can be attached to the foam structure with a surgical hammer.

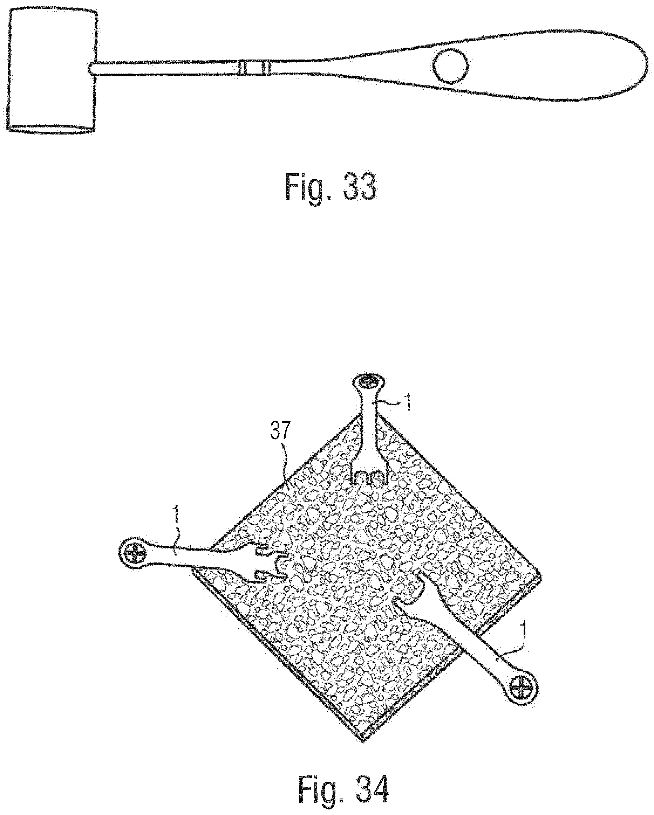

Fig. 33 shows a chirurgical hammer for attaching the surgical fixation device to a foam structure.

Fig. 34 shows three different surgical fixation devices, all fixed to a foam structure.

In summary, by considering the specific design guidelines of this novel fixation device a sufficient anchoring of the hooks into the foam-like structure can be achieved.

### [Solutions used prior to the disclosure]

Fixation devices in the CMF area are usually developed to fixate solid implant structures (for example screws and plates). Currently there are no existing metal foam implants on the CMF market. Plastic (Medpor) implants are fixated with screws generating increased profile height or they do not attain the same stability. Using a titanium foam implant as an inlay to reconstruct bone defects in the midface and at the cranial area is a novel and innovative technology. Therefore, no foam-specific fixation devices existed before the present disclosure in the CMF sector. Also, in other ranges of indications no foam-specific fixation solutions could be identified.

### [Exemplary advantages]

Since there are no existing metal foam implants in the CMF market currently there are no structure-specific fixation solutions existing. The novel implant fixation device is specifically designed for the attachment of an open-porous metal foam structure. During the constructive design the dimensions of the foam-structure are considered. The logic behind is documented so that the solution could be easily adapted to e.g. a different pore width of the foam. With this design a proper stability can be reached. This is an important advantage in comparison to other fixation devices which are usually designed for the fixation of solid materials/implants. Reaching an interlocking mechanism between the hooks and the porous structure while considering the specific structure of the material shapes this novel product into a unique and innovative solution.

Another advantage is the good usability of the hook plate. Attaching the hook plate to the foam material is easy to use and time efficient. A simple instrument such as a hammer can be used to attach the plate to the foam.

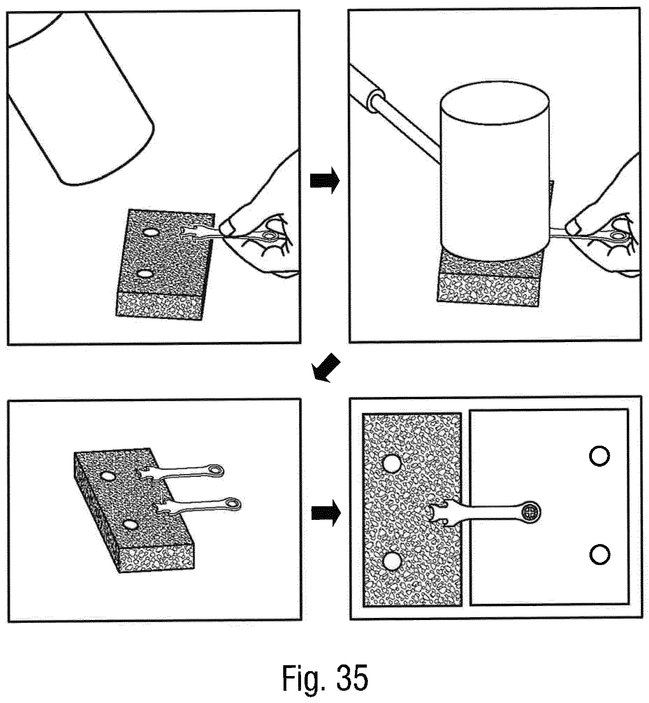

Fig. 35 shows four steps of a method for attaching the surgical fixation device (here: hook-plate) to a foam structure (here: metal foam), using for example a surgical hammer, and the attached combination of both to a bone.

From a usability perspective a higher stability and a faster attachment compared to the prior art is achieved. The product costs can be lower compared to existing solutions. Only equal or superior behavior of the product is known so far.

### [Further technical aspects]

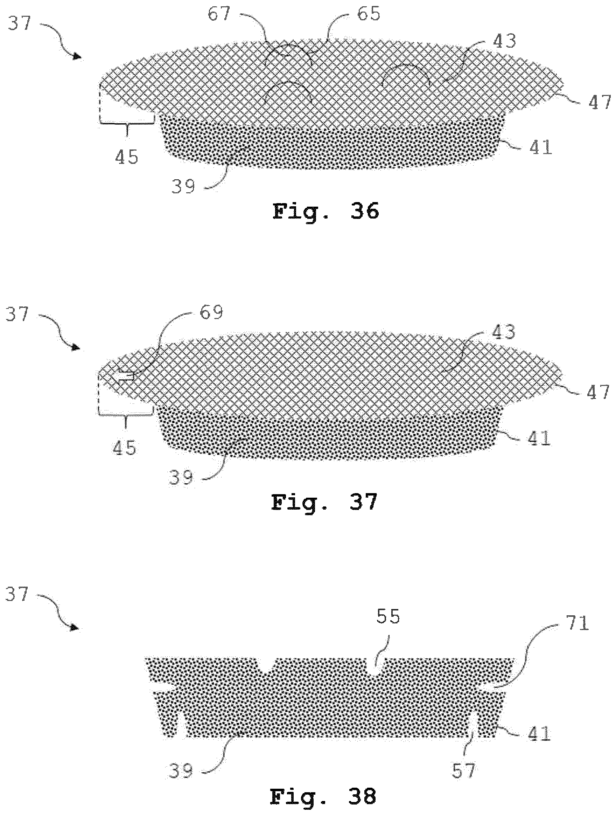

Fig. 36 shows a foam structure 37 in combination with protruding eyes 65. Here, the eyes 65 protrude from the fixation device structure 43 already described with reference to Fig. 7. However, such eyes 65 may additionally or alternatively protrude from the porous body 39. Moreover, such eyes 65 may generally be provided in any of the variants of Figs. 6 to 14.

In this illustration, there are three eyes 65. However, there may generally be one or more, i.e., two, three, four etc., eyes 65. Each eye 65 approximately forms a semicircle in a plane that is orthogonal to the surface from which the eye 65 protrudes. However, this shape and inclination of the eye 65 may differ. The key aspect of the eye 65 is its opening 67. By providing such an opening 67, the surgeon is able to use it as an additional attachment point, e.g., for suturing muscle to it. Of course the eye 65 can be a separate structure that is attached to the foam structure 37 or integrally formed with the foam structure 37, e.g., by 3D-printing.

Fig. 37 shows a foam structure 37 in combination with an alignment indication 69, which helps the surgeon insert and align the foam structure 37 properly during surgery. The indication 69 may be provided on the fixation device structure 43 and/or the porous body 39. In particular, the indication 69 may be provided at an edge and/or edge portion 45 of the fixation device structure 43 or the foam structure 37. Structurally, the indication 69 may be formed as an indentation or as a protrusion to allow the surgeon to feel the indication 69. Further, several indications 69 may be provided, at least some of which may differ from each other, distributed on the surface of the foam structure 37. Here, the indication 69 is an arrow, but the shape of the indication 69 is not limited thereto and may have other shapes.

Fig. 38 shows a further modification of the modification of the foam structure 37 shown in Fig. 14. In addition to the top recesses 55 and the bottom recesses 57, this illustration shows lateral recesses 71 on both sides of the foam structure 37. Of course, the lateral recesses 71 may be provided without the top recesses 55 and/or the bottom recesses 57. Further, each of the recesses 55, 57 and 71 may be dimensioned and shaped such that it is capable of holding bone chips, for example by providing a recess that widens towards the inside direction. Thereby, bone chips can be pushed into the recess but will cant therein and become less likely to escape the recess during handling of the foam structure 37.

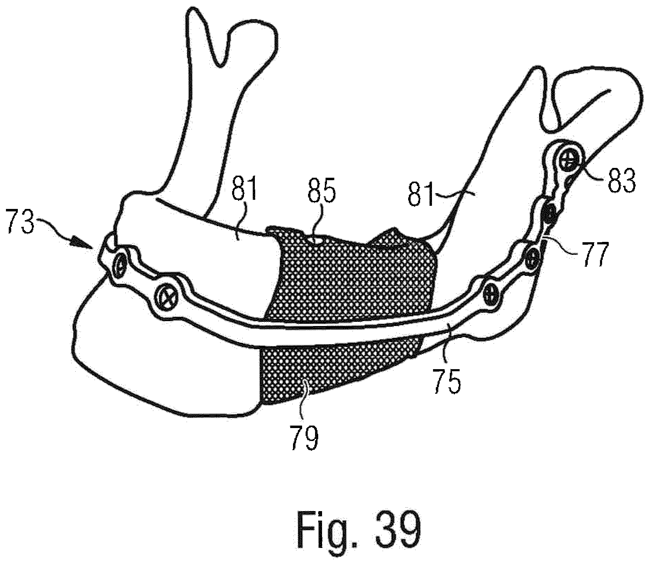

Fig. 39 shows a mandibular implant 73 with a bone attachment structure 75. The attachment structure 75 comprises a linearly extending bone plate 77 with an arbitrary number of screw holes 83 and a mesh-type enclosure 79 for a porous body as generally presented herein (not shown in the perspective of Fig. 39). The enclosure 79 wraps around the porous body. The enclosure 79 generally has the shape of a missing mandibular bone segment, but could have any other shape in other variants.

In the present use case, the porous body extends between two distant ends of the enclosure 79 to contact bone portions 81 defining a bone gap therebetween, and to allow bone ingrowth within the enclosure 79. Of course, instead of providing the enclosure 79, the porous body may be directly connected with the bone plate 77. All of these components may be manufactured as one single piece / monolithically, e.g., by 3D-printing, or may be made separately and connected to each other at a later stage prior to surgery by one of the known connection methods, e.g., welding, screws etc. In the enclosure 79 with porous body or in the porous body alone, there may be provided a dental implant recess 85. The recess 85 may be formed as an indentation in the enclosure 79 as shown here.

In this case as well as in any other case of the present disclosure, where soft tissue retraction and implant exposure might become an issue, the attachment structure 43, 73 may be covered with a Medpor barrier or hydrogel (resorbable or non-resorbable) or autologous tissue (fat, bone chips).

In this case as well as in any other case, where infection may occur, it is possible to add antibiotics to the HA coating.

For not load-bearing use cases, the attachments structure and the porous body may be connected by a resorbable yarn or wire.

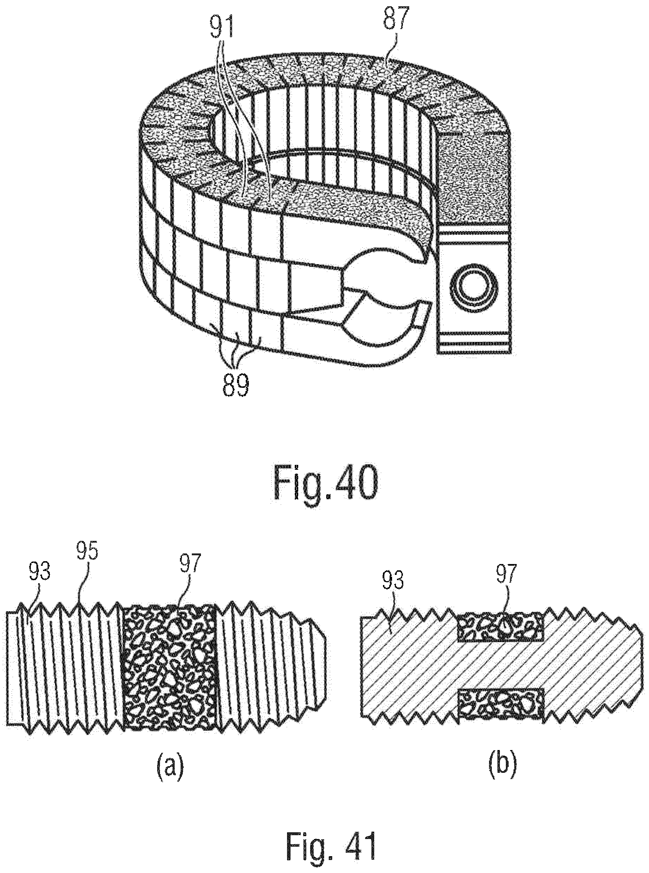

Fig. 40 shows a porous body 87 to be used for spinal interventions. In this example the porous body 87 is made from multiple porous sub-bodies 89, which are provided in a row and connected in such a manner as to be capable to be inclined relative to each other. For this purpose the sub-bodies 89 may be portions of a monolithic porous body 87 connected by interconnecting sections 91 with increased flexibility compared the rest of the body 87. These interconnecting sections 91 may be provided by merely weakening the otherwise uniform porous body 91 locally. Alternatively or additionally, hinges made of the same or different material may be provided between the separately formed porous sub-bodies 89.

Fig. 41 shows a side view (a) and a cross-section (b) of a system with a surgical fixation device 93 (e.g., a spinal screw) provided with an outer thread 95. One portion of the surgical fixation device 93 comprises a porous body 97 located in a recess thereof. The porous body may be covered by a mesh or other stabilizing structure on its side facing bone.

In the exemplary variant of Fig. 41, the porous body 97 axially extends between two thread sections of the outer thread 95. However, it may extend any other way as well. Alternatively or additionally, the porous body 97 may comprise a thread. Alternatively or additionally, the porous body 97 can extend axially through the surgical fixation device 93 to be exposed at one or both axial ends of the surgical fixation device 93. In this system, the porous body 97 is ring shaped and coaxial with the surgical fixation device 93. Alternatively or additionally, the porous body 97 may be shaped as a helix on the outer side of the surgical fixation device 93. Such a helix may form at least one of the threads of the outer thread 95. Again, the surgical fixation device 93 and the porous body 97 of the foam structure may be manufactured in a monolithic manner using 3D-printing.

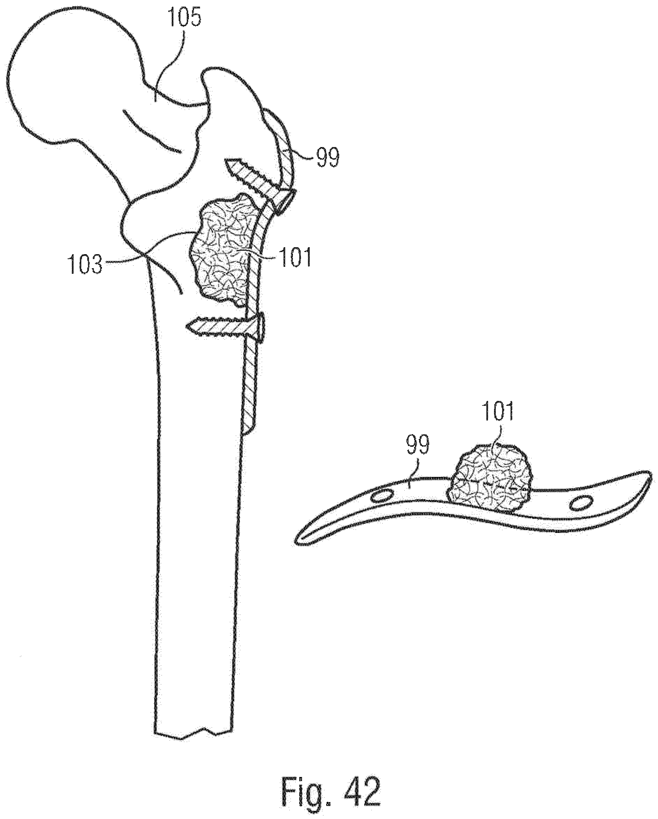

Fig. 42 shows a bone plate system comprising a surgical fixation device 99 (configured as a bone plate with dedicated screw holes) and a foam structure 101 attached thereto. The fixation device 99 and/or the foam structure 101 are custom made to correspond to a bone cavity 103 and to the outer shape of the bone 105 with the bone cavity 103. In some variants, such as in the scenario of Fig. 42, the bone 105 is a femur.

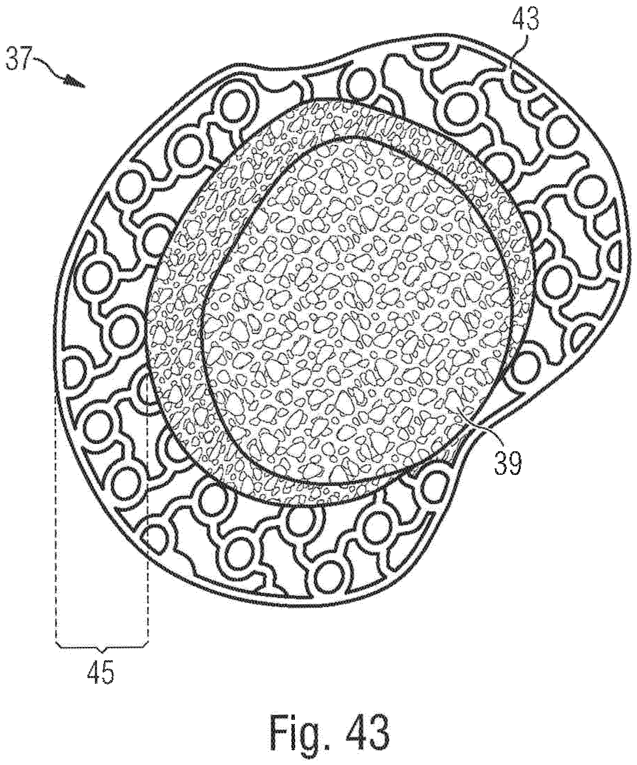

Fig. 43 shows a modification of the modification of the system illustrated in Fig. 7, 11 or 13, where the edge portion 45 of the attachment structure 43 is non-uniform and comprises a varying, patient-specific extension from the porous body 39 along the circumference of the porous body 39 (see also Fig. 14C). The system illustrated in Fig. 43 may be configured to close a cranial bone defect. As can be gathered from Fig. 43, the porous body 43 has inclined (e.g., frustro-conical) side surfaces so as to match correspondingly inclined lateral bone surfaces of the cranial bone defect. The inclination helps to increase the contact surface between the porous body 43 and the adjacent bone.

The following examples are also encompassed by the present disclosure and may fully or partly be incorporated into embodiments. The reference signs presented in the examples only serve for a better understanding and are not intended to have a limiting effect.
1. A system comprising a foam structure (37) and a surgical fixation device (1, 43, 93, 110) for attaching the foam structure (37) to bone, the foam structure (37) comprising:
   a porous body (39) made of at least one biocompatible implant material, wherein the porous body (39) is coated with a coating, which is capable of stimulating bone ingrowth.
2. The system of example 1, wherein
   the porous body (39) and, optionally, the fixation device (1, 43), is 3D-printed, for example in a monolithic manner.
3. The system of one of examples 1 to 2, wherein
   the surgical fixation device (1, 43) comprises a stabilizing structure (1, 43) that is, for example monolithically, provided on one side of the foam structure (37).
4. The system of example 3, wherein
   the stabilizing structure (1, 43) laterally extends over the foam structure (37) to define one or more bone attachment regions, wherein, as an option, an attachment opening (11, 49), in particular a screw hole (11), is formed in each bone attachment region.
5. The system of example 3 or 4, wherein
   the stabilizing structure (1, 43), comprises a plurality of geometric entities that are at least in regions spaced apart from each other.
6. The system of example 5, wherein
   the geometric entities comprise straight or non-straight lines, wherein, as an option, the lines form a mesh or extend substantially parallel to each other.
7. The system of one of examples 5 to 6, wherein
   the geometric entities have at least one dimension that exceeds an average pore size and/or that exceeds 0.5 mm, in particular 1 mm or 1.5 mm.
8. The system of one of examples 1 to 7, comprising:
   an attachment structure (43), which is monolithic with or fixed to the porous body (39), wherein, as an option, the fixation device constitutes or comprises the attachment structure (43).
9. The system of one of examples 1 to 7, wherein
   one side of the porous body (39) comprises a closed layer and/or the porous body (39) comprises recesses (55, 57) on one side or on opposite sides of the porous body (39).
10. The system of one of examples 1 to 9, wherein
   the coating is a hydroxyapatite coating.
11. The system of one of examples 1 to 10, wherein
   the coating has a thickness between 1 µm and 200 µm, in particular between 3 µm and 100 µm.
12. The system of one of examples 1 to 11, wherein
   at least more than 50% of the coated pores have an internal dimension ranging between 0.3 mm and 1 mm, in particular between 0.4 mm and 0.6 mm.
13. The system of one of examples 1 to 12, wherein
   the fixation device (43) is a bone screw or a bone plate, that optionally covers the foam structure (37).
14. The system of one of examples 1 to 13, wherein
   the foam structure (37) and the surgical fixation device (1, 43, 110) are made of the same material and with a thickness of the surgical fixation device (1, 42, 110) of 1 mm or less at an attachment region with the foam structure (37), and
   the foam structure (37) comprises a thickness in the range of 11.3 mm to 0.3 mm as well as a porosity in the range of 1.4 to 0.8.
15. The system of one of examples 1 to 14, wherein
   the foam structure (37) comprises a thickness in the range of 3.4 mm to 0.3 mm as well as a porosity in the range of 1.4 to 0.8.
16. The system of one of examples 1 to 15, wherein
   the coating comprises antibiotics.
17. The system of one of examples 1 to 16, wherein
   at least one of the foam structure (37) and the surgical fixation device (1, 43, 110) comprises one or more protruding eyes (65).
18. The system of one of examples 1 to 17, wherein
   the foam structure (37) comprises one or more lateral recesses (71).
19. The system of one of examples 1 to 18, wherein
   at least one of the foam structure (37) and the surgical fixation device (1, 43, 110) comprises at least one alignment indication (69).
20. The system of any one of examples 1 to 19, wherein
   the foam structure (37) and the surgical fixation device (1, 43, 110) are connected by a resorbable connector.
21. The system of any one of examples 1 to 20, wherein
   the foam structure (37) comprises multiple porous sub-structures (89) capable of being inclined relative to each other.
22. The system of any one of examples 1 to 21, wherein
   the surgical fixation device (93) is provided with an outer thread (95) and comprises a recess filled with by at least a portion of the foam structure (97).

## Claims

1. A surgical foam structure (37) comprising an open-porous body (39) made of at least one biocompatible implant material,
wherein the porous body (39) is dimensioned such that it fulfills at least one of the following:
- a porosity is in the range of 0.81 to 0.91 %; and
- an average pore diameter is in the range of 1.10 to 1.41 mm.

2. The surgical foam structure (37) of claim 1, wherein
the porous body (39) is coated with a coating capable of stimulating bony ingrowth into the pores of the porous body (39).

3. The surgical foam structure (37) of claim 2, wherein
the coating is a hydroxyapatite coating.

4. The surgical foam structure (37) of claim 2 or 3, wherein
at least more than 50% of the coated pores have an internal dimension ranging between 0.3 mm and 1 mm, in particular between 0.4 mm and 0.6 mm.

5. The surgical foam structure (37) of one of claims 1 to 4, wherein
the pores have the shape of an octaeder.

6. The surgical foam structure (37) of one of claims 1 to 5, wherein
the porous body (39) comprises multiple porous sub-structures (89) capable of being inclined relative to each other.

7. The surgical foam structure (37) of one of claims 1 to 6, wherein
the porous body (39) is 3D-printed, for example in a monolithic manner.

8. The surgical foam structure (37) of one of claims 1 to 7, wherein
the foam structure (37) comprises one or more protruding eyes (65).

9. The surgical foam structure (37) of one of claims 1 to 8, wherein
the foam structure (37) comprises one or more lateral recesses (71).

10. The surgical foam structure (37) of one of claims 1 to 9, wherein
the foam structure (37) comprises one or more top recesses (55) and/or one or more bottom recesses (57).

11. The surgical foam structure (37) of claim 9 or 10, wherein
the recesses (55, 57, 71) widen towards the inside direction.

12. The surgical foam structure (37) of one of claims 1 to 11, wherein
the foam structure (37) comprises at least one alignment indication (69).

13. The surgical foam structure (37) of one of claims 1 to 12, wherein
the porosity of the foam structure (37) varies throughout the foam structure (37), wherein, optionally, it follows a specific pattern.

14. The surgical foam structure (37) of one of claims 1 to 13, wherein
the porosity of the foam structure (37) decreases or increases from a body edge (41) of the foam structure (37) towards a center of the foam structure (37).

15. A system comprising:
the surgical foam structure (37) of one of claims 1 to 14, and
a surgical fixation device (1, 43, 93, 110) for attaching the foam structure (37) to bone.
